# EUROPÄISCHE PATENTSCHRIFT

(11) **EP 2 563 370 B1**
(45) Veröffentlichungstag und Bekanntmachung des Hinweises auf die Patenterteilung: **17.12.2014**
(21) Anmeldenummer: 11716415.2
(22) Anmeldetag: 28.04.2011
(51) Int. Cl.: A61K 31/66, A61K 31/685, A61K 31/704, A61P 3/04, A61P 43/00, A61Q 19/06, A61K 8/63, A61K 8/55

(54) **VERWENDUNG EINER PHOSPHOLIPID UND GLYCYRRHIZINSÄURE ENTHALTENDEN ZUSAMMENSETZUNG ZUR ENTFERNUNG VON SUBKUTANEN FETTANSAMMLUNGEN DURCH SUBKUTANE LIPOLYSE**
USE OF A COMPOSITION CONTAINING PHOSPHOLIPIDS AND GLYCYRRHIZINIC ACID FOR REMOVING SUBCUTANEOUS FAT ACCUMULATIONS BY MEANS OF SUBCUTANEOUS LIPOLYSIS
UTILISATION D'UNE COMPOSITION CONTENANT UN PHOSPHOLIPIDE ET UN ACIDE GLYCYRRHIZIQUE POUR L'ÉLIMINATION D'ACCUMULATIONS DE GRAISSE SOUS-CUTANÉE PAR LIPOLYSE SOUS-CUTANÉE

(30) Priorität: 29.04.2010 DE 102010028365
(43) Veröffentlichungstag der Anmeldung: 06.03.2013
(73) Patentinhaber: Lichtblick GmbH, 48317 Drensteinfurt (DE)
(72) Erfinder: GUNDERMANN, Karl-Josef, 50933 Köln (DE); BRANDL, Dirk, 48324 Albersloh (DE)
(74) Vertreter: Gille Hrabal
(86) Internationale Anmeldenummer: PCT/EP2011/056721
(87) Internationale Veröffentlichungsnummer: WO 2011/135020

(56) Entgegenhaltungen:
- WO-A2-2007/020505
- DE-A1- 10 349 979
- DE-A1- 10 361 067
- ARTEGODAN: "Lipostabil", 20081001, [Online] 1. Oktober 2008 (2008-10-01), Seiten 1-2, XP007918845, Gefunden im Internet: URL:http://www.fachinfo.de/data/fi/jsearch ?praep> [gefunden am 2011-06-06]

## Beschreibung

Die Erfindung betrifft die Verwendung einer Zusammensetzung auf der Grundlage von Phospholipiden und Glycyrrhizinsäure oder einem Salz der Glycyrrhizinsäure, und deren in der Medizin anwendbaren Formen, zur Behandlung von adipösen Einlagerungen unter der Haut verschiedenster Erscheinungsformen, wie z.B. subkutane Fettverteilungsstörungen, und zur Regression von diätresistenten Fettpolstern.

### Stand der Technik

Im Stand der Technik werden bisher chirurgische Methoden verwendet, um subkutane Fettansammlungen oder Wucherungen der Fettzellen wie Lipome oder Lipödeme zu behandeln. Derartige Behandlungsmaßnahmen führen zu den bekannten Komplikationen oder Risiken, verursacht durch Narkose, lokale Reaktionen und mögliche Infektionen. Stationäre Klinik-Aufenthalte sind unter solchen Umständen häufig nicht zu vermeiden. Um solche Eingriffe zu vermeiden, wird nach nicht-operativen Alternativen zur Entfernung von subkutanen Fettansammlungen gesucht.

Es wurden bereits verschiedene phospholipidhaltige Präparate entwickelt, die als Injektion an Patienten verabreicht werden. Wässrige Zubereitungen, enthaltend mindestens ein Phospholipid, sind für verschiedene Anwendungen bekannt. Diese Systeme werden beispielsweise im kosmetischen Bereich oder für die Herstellung von pharmazeutischen Produkten eingesetzt. Häufig bilden diese Systeme Mizellen oder Liposome aus, die in ihrem Inneren eine wässrige Phase enthalten.

Die US 2005/143347 A1 sowie das dazugehörige Prioritätsdokument DE 103 61 067 A1 offenbaren eine wässrige Zubereitung enthaltend mindestens ein Phospholipid und/oder mindestens eine Gallensäure und eine Fettabbau unterstützende Komponente wie Riboflavin und Wasser, die sich zur Herstellung von Arzneimitteln zur Entfernung von subkutanen Fettansammlungen und zur Regression der Fettpolster eignen. Als kommerziell erhältliche Präparate werden dort Essentiale^{®} N i.V. (Rote Liste, März 2003) und Lipostabil^{®} N i.V. genannt.

Essentiale^{®} N i.V. ist in der EP 0 615 746 A1 als eine wässrige Zubereitung beschrieben, enthaltend Phospholipide aus Sojabohnen, Gallensäure, Riboflavin, alpha-Tocopherol, Ethanol und Wasser. Die Wirkstoffzusammensetzung wird in einem mizellaren System verarbeitet, wobei die Mizellen einen Durchmesser von 30 nm bis 100 nm aufweisen. In der EP 0 615 746 A1 ist die Verwendung der Glycyrrhizinsäure nicht beschrieben. Dieses Präparat wird intravenös verabreicht, um unter anderem eine Verfettung der Leber, bei der es sich um einen übermäßigen Fettgehalt des Leberparenchyms (Fettablagerung in Tröpfchenform) handelt, zu behandeln.

In der DE 103 61 067 A1 wird die wässrige Zubereitung der Arzneiform Essentiale^{®} N i.V. enthaltend mindestens ein Phospholipid und/oder mindestens eine Gallensäure und eine Fettabbau unterstützende Komponente und Wasser zur Herstellung eines Arzneimittels zur Entfernung von subkutanen Fettansammlungen offenbart. Auch hier wird der Zubereitung keine Glycyrrhizinsäure beigefügt.

Lipostabil^{®} N i.V. enthält Phospholipide aus der Sojabohne, DL-alpha-Tocopherol, 7-Desoxycholsäure, Alkohole, weitere Hilfsstoffe und Wasser. Allerdings enthält die Wirkstoffzusammensetzung dieses Präparates keine Glycyrrhizinsäure. Durch die subkutane Injektion von Lipostabil^{®} N i.V. werden Fetteinlagerungen, wie sie bei übergewichtigen Menschen unter den Augen, am Bauch oder an Hüften auftreten, entfernt.

Es gibt noch weitere Zubereitungen, die auf einem Phospholipid basieren und zur subkutanen Injektion zwecks Lipolyse der Fettansammlungen verwendet werden, welche in der WO 2008/113421, DE 10 2007 015 701, US 2005/143347 A1 und US 2005/0089555 A1 beschrieben sind.

Nachteile der Verwendung dieser Präparate des Standes der Technik für eine subkutane Lipolyse sind unter anderem Schwellungen, Hämatome (Blutergüsse), Schmerzen in der Behandlungsregion und Missempfinden wie ein brennendes Gefühl und Juckreiz an der Injektionsstelle nach der Behandlung, insbesondere aber ein Absterben von Zellen durch Schädigung der Zellstruktur und der Membranintegrität (Zellnekrose).

In dem Bestreben, wirksame Verbindungen zur nicht-operativen Entfernung von subkutanen Fettansammlungen ohne die oben genannten Nachteile zu finden, wurde nun überraschenderweise gefunden, dass die Zusammensetzung, welche in der RU 2 133 122 C1 zur intravenösen und oralen Behandlung von akuten und chronischen Lebererkrankungen sowie Störungen des Lipidstoffwechsels bei Arteriosklerose und begleitenden Erkrankungen offenbart wird, für eine subkutane Lipolyse, auch Adipozytolyse genannt, geeignet ist.

Bei der erfindungsgemäßen Verwendung dieser Zusammensetzung, die mindestens ein Phospholipid, bevorzugt ein Phosphatidylcholin, und Glycyrrhizinsäure oder deren Salz enthält, werden die zuvor beschriebenen Nachteile der Präparate des Standes der Technik, die für eine subkutane Lipolyse verwendet werden, vermindert und/oder gänzlich behoben.

Der besondere Vorteil der erfindungsgemäßen Verwendung der beanspruchten Zusammensetzung zur subkutanen Lipolyse von Fettansammlungen ist die deutliche Reduzierung bis zur vollständigen Verhinderung der Schädigung der Zellstruktur sowie der Membranintegrität der adipösen Gewebezellen (Adipozyten). Bei der erfindungsgemäßen Behandlung von subkutanen Fettgewebsstörungen mit der beanspruchten Zusammensetzung wird das Auftreten von Nekrose deutlich vermindert oder gänzlich verhindert.

Durch die erfindungsgemäße Verwendung kommt es zum Abbau von Depotfett im Körper des behandelten Fettgewebes durch Lipolyse, ohne dass eine Destruktion der Zellmembran eintritt.

Es werden deutlich geringere Nebenwirkungen bis zu einem vollständigen Ausbleiben von Beschwerden wie Schwellungen, Rötungen, Brennen, Juckreiz und allgemeine Schmerzen und Überempfindlichkeit beobachtet.

Die erfindungsgemäß verwendete Zusammensetzung beinhaltet als Hauptbestandteile ein Phospholipid, Glycyrrhizinsäure oder deren Salz und gegebenenfalls mindestens einen Hilfsstoff.

Glycyrrhizinsäure, die aus einem Extrakt von Pflanzen der Gattung Glycyrrhiza, den Süßhölzern (z.B. Glycyrrhiza glabra) gewonnen werden kann, oder ihre Salze, insbesondere Natrium- oder Ammoniumglycyrrhizinat, werden im Stand der Technik als Absorptionsverstärker für den Transport von z.B. Peptidhormonen über die Schleimhautmembran in der EP 03 27 756 A beschrieben. Auch die Verstärkung der Absorption von Polypeptiden durch Glycyrrhizinsäure in transvaginalen Zubereitungen wird in der US 5 238 917 A beschrieben.

Darüber hinaus ist die Glycyrrhizinsäure als antibakterieller und entzündungshemmender Zusatzstoff und Emulgator im Stand der Technik aus der US 2004-076652 A, US 2009-169588 A, US 2007-053852 A, WO 08/046791 A, WO 08/046795 A, WO 05/037239 A, EP 1 676 561 A und der JP 2006/137 670 bekannt.

Die besondere Funktion der Glycyrrhizinsäure beim Transport von Molekülen über die Zellmembran unterstützt den besonderen Vorteil der schnellen Wirkungsweise der erfindungsgemäßen Verwendung der beanspruchten Zusammensetzung. Durch die Glycyrrhizinsäure kann das Phospholipid besonders gut und schnell in die adipösen Gewebszellen der Subkutis gelangen, wodurch die zuvor beschriebenen Nachteile wie Nekrose vermindert werden oder gar nicht auftreten. Die beschriebenen Schwellungen, Hämatome, Schmerzen und Missempfindungen nach einer Behandlung mit einem Präparat des Standes der Technik, insbesondere solcher, die Gallensäuren oder Salze dieser enthalten, werden durch die Kombination mit der Glycyrrhizinsäure gelindert oder treten gar nicht auf. Diese verbesserte Verträglichkeit wird durch die entzündungshemmende und antibakterielle Wirkung der Glycyrrhizinsäure unterstützt.

Folglich zeichnet sich die vorliegende Erfindung dadurch aus, dass die beanspruchte Kombination aus mindestens einem Phospholipid, bevorzugt einem Phosphatidylcholin und Glycyrrhizinsäure oder ihres Salzes im Vergleich zu den Zusammensetzungen der Präparate Essentiale^{®} und Lipostabil^{®} aus dem Stand der Technik wesentlich geringere bis keine zellschädigende Wirkung und eine verbesserte Verträglichkeit hat.

Die Erfindung betrifft daher die Verwendung einer Zusammensetzung, enthaltend
a) mindestens ein Phospholipid;
b) Glycyrrhizinsäure, oder
   ein Salz der Glycyrrhizinsäure und
c) ggf. Hilfsstoffe
   wobei
   - der Gesamtgehalt an den Phospholipiden und der Glycyrrhizinsäure oder deren Salzen 2-80 Gewichtsprozent aufweist, und
   - das Gewichtsverhältnis zwischen Phospholipiden und der Glycyrrhizinsäure oder deren Salze von 30:1 bis 0,5:1 beträgt,
      zur Herstellung eines Arzneimittels zur therapeutischen Behandlung von subkutanen Fettgewebserkrankungen.

Die Erfindung betrifft ferner die Verwendung zur Zersetzung und Rückbildung von Fettgewebsgeschwülsten.

Die Erfindung betrifft ferner die Verwendung zur Behandlung von subkutanen Fettgewebserkrankungen wie Lipödeme, Lipome, Morbus Dercum, Madelung'schen Fetthals oder Lipomatosis der Bauchdecke.

Die Erfindung betrifft ferner die nicht-therapeutische Verwendung einer Zusammensetzung, enthaltend
a) mindestens ein Phospholipid;
b) Glycyrrhizinsäure, oder
   ein Salz der Glycyrrhizinsäure und
c) ggf. Hilfsstoffe
   wobei
   - der Gesamtgehalt an den Phospholipiden und der Glycyrrhizinsäure oder deren Salzen 2-80 Gewichtsprozent aufweist, und
   - das Gewichtsverhältnis zwischen Phospholipiden und der Glycyrrhizinsäure oder deren Salze von 30:1 bis 0,5:1 beträgt,
      zur Behandlung von subkutanen Fettansammlungen.

Die Erfindung betrifft ferner die nicht-therapeutische Verwendung zur Entfernung von subkutanen Fettansammlungen, insbesondere mit lokaler Störung der Fettverteilung.

Die Erfindung betrifft ferner die nicht-therapeutische Verwendung zur Behandlung von Dermatopanniculosis deformans, Pseudogynäkomastie, Buffalo Hump bei HIV Patienten, Cellulite, Xanthelasmen oder unspezifische subkutane Fettdepots.

Die Erfindung betrifft ferner die Verwendung der obigen Zusammensetzung welche Phosphatidylcholin als Phospholipid enthält.

Die Erfindung betrifft ferner die Verwendung, wobei die Zusammensetzung Phosphatidylcholin tierischen oder pflanzlichen Ursprungs enthält.

Die Erfindung betrifft ferner die Verwendung wobei die Zusammensetzung Glycyrrhizinsäure oder Kalium-, Natrium-, Ammonium- oder Magnesiumsalze der Glycyrrhizinsäure enthält.

Die Erfindung betrifft ferner die Verwendung wobei die Zusammensetzung einen Zucker, insbesondere Glukose und Maltose und/oder ihre Derivate, Mannit, Sorbit oder Milchzucker als Hilfsstoff enthält.

Die Erfindung betrifft ferner die Verwendung wobei das Phospholipid 15 bis 98 Gew.-%, bevorzugt, 30 bis 98 Gew.-%, mehr bevorzugt 50 bis 98 Gew.-%, besonders bevorzugt 75 bis 98 Gew.-%, am meisten bevorzugt 75 bis 90 Gew.-% Phosphatidylcholin enthält.

Die Erfindung betrifft ferner die Verwendung wobei die Zusammensetzung in trockener Form in einem geeigneten Lösungsmittel gelöst wird.

Die Erfindung betrifft ferner die Verwendung wobei die Zusammensetzung in trockener Form bevorzugt als Lyophilisat, erzielt durch Gefriertrocknung, eingesetzt wird.

Die Erfindung betrifft ferner die Verwendung wobei die Zusammensetzung in Form von einer Lösung eingesetzt wird.

Die Erfindung betrifft ferner die Verwendung wobei die Zusammensetzung physiologisch geeignete Lösungsmittel umfassend Wasser, physiologische Kochsalzlösung, Glucose, ein Monohydroxy-Alkohol wie Ethanol, 2-Propanol, n-Propanol, Polyhydroxy-Alkohole wie Glyzerol und/oder Propandiol, Polyglykol wie Polyethylenglykol und/oder Miglyol, Glycerinformal, Dimethylisosorbitol, natürliche und synthetische Öle und/oder Äther enthält.

Die Erfindung betrifft ferner die Verwendung wobei die Applikation der Zusammensetzung durch subkutane, intraperitoneale, intramuskuläre oder intravenöse Injektion erfolgt.

Die Erfindung betrifft ferner die Verwendung wobei zur Applikation der Zusammensetzung ein Verfahren ausgewählt aus der Gruppe umfassend lontophorese, Elektroporation, Microporation oder Phonophorese eingesetzt wird.

Die Applikation der erfindungsgemäßen Zubereitung erfolgt in der Form von Cremes, Salben, Gelen, Hydrogelen, Lotionen, Pasten, Lyophilisat und Lösungen. Bevorzugt ist die wässrige Zubereitung in Form von verschiedenen Lösungen.

Durch die erfindungsgemäße Verwendung der Zusammensetzung können die oben genannten Risiken und Nebenwirkungen einer operativen Behandlung oder einer subkutanen Behandlung mit einem Präparat aus dem Stand der Technik, insbesondere solcher enthaltend Deoxycholsäure und ihre Salze, umgangen werden. Zudem ist die ambulante Behandlung für den Patienten angenehmer und auch kostengünstiger im Vergleich zur operativen Behandlung.

Die oben beschriebene hohe therapeutische Effizienz, die sich durch den schnellen Abbau des Fettgewebes der Zusammensetzung zeigt, ist mit dem Synergieeffekt des Zusammenspiels der erfindungsgemäßen Kombination aus einem Phospholipid und/oder einer Glycyrrhizinsäure oder eines ihrer Salze verbunden. Die erfindungsgemäße Verwendung der hier beanspruchten Zusammensetzung zeichnet sich durch deutlich abgeschwächte Nebenwirkungen oder zum Teil durch Ausbleiben einiger der zuvor beschriebenen Nebenwirkungen aus.

Subkutane Fettverteilungsstörungen sind Abweichungen im Fettgewebe im Körper von Menschen und Säugetieren, die als genetisch oder ernährungsbedingtes Depotfett in Form lokalisierter Fettpolster auftreten und als ästhetisch störende kritische Zonen, insbesondere Bauch, Gesäß, Hüften, Knie, Waden, Oberschenkel, Oberarme, Kinn, Wangen angesehen werden können. Es kann sich dabei auch um gutartige Wucherungen der Fettzellen wie Lipome (dystopische Proliferation) handeln.

Unter Fettgewebserkrankungen im Sinne der Erfindung werden beispielsweise folgende Erkrankungen verstanden: Lipome (Fettgewebsgeschwülste), dies sind gutartige, langsam wachsende, meist kugelige, eventuell gestielte (= L. pendulum) oder gar zottige (= L. arborescens, beispielsweise der Gelenkzotten) mesenchymale Geschwülste aus - vergrößerten - Fettgewebszellen, bevorzugt im Unterhautzellgewebe, eventuell zentral verknöchernd (= L. ossificans), verschleimend (= L. myxomatodes) oder verkalkend (= L. petrificans), auch mit vermehrter Bindegewebs- und Kapselbildung (= L. fibrosum), Blutgefässneubildung (= L. teleangiectodes), selten maligne entartend (= L. sarcomatodes, Liposarkom). Sie sind als krankhaft einzustufen, da sie wachsen und ihre bindegewebige Hülle an sich schmerzhaft sein kann, ebenso wie die von ihnen ausgehende Kompression auf Blutgefäße, die Nervenschmerzen verursachen kann. Hierunter fällt auch die multiple Lipomatosis, die zu einer gehäuften Ansammlung von Lipomen beim Patienten führt.

Morbus Dercum, genannt Lipomatosis dolorosa ist eine Sonderform der hypertrophen Proliferation von Fettgewebe, welches sich zwischen der dermalen Fettfaszie (Kampa'sche Fettfaszie) und der Unterseite der Dermis befindet. Durch hormonelle Einflüsse kommt es zur verstärkten Wasserbindungskapazität dieser Fettzellen, die selbst wiederum durch Druckphänomene Lymphbahnstauungen im Bereich der initialen farnkrautartigen Lymphgefäße herbeiführen, wodurch zusätzliche Kompressions- und Irritationseinflüsse auf die peripheren sensiblen Nerven ausgeübt werden, so dass diese Patienten eine extrem schmerzhafte Berührungsempfindlichkeit aufweisen. Im Verlaufe von mehreren Jahren bis Jahrzehnten bilden sich unregelmäßige, unter der während des Alterungsprozesses dünner werdenden Dermis disseminiert lokalisierte Fettknötchen, welche teils schmerzhaften und stark dysästhetischen Charakter haben.

Der Madelung'sche Fetthals (Lanois-Bensaude-Syndrom) ist eine fettgewebsproliferierende Fettgewebsentzündung, bei der es neben einer dystrophen Fettgewebstumorausbildung auch zu einer narbenartigen Bindegewebsverdichtung im Subkutanraum kommt. Hierbei können operative Vorgehensweisen oft nur Teilerfolge erzielen, da essentielle anatomische Strukturen in diesem Prozess mit eingeschlossen sind, und die Erkrankung sich im wesentlichen im Kopf-, Hals- und Schulterbereich manifestiert.

Das Lipödem ist eine schmerzhafte Schwellung des Fettgewebes, die besonders an den Unterschenkeln von Frauen auftritt und mit zunehmendem Alter einen fortschreitenden Verlauf bzw. Charakter aufweist.

Als Regression der Lipolyse wird also die hydrolytische Spaltung des Fettgewebes und Rückbildung durch Mobilisierung des proliferierten Fettbereichs verstanden.

Ein Xanthelasma ist eine gelbliche Ansammlung von Fett unter den Augen.

HIV Patienten haben häufig Störungen von Fettgewebsansammlungen, die durch die Medikamentierung nach dem Stand der Technik auftreten, beispielsweise der Buffalo Hump genannte Stiernacken bei dieser Patientengruppe. Immunsystem geschwächten Patienten ist in der Regel eine operative Entfernung nicht zuzumuten, weshalb die Fettansammlungen verbleiben, und die Patienten äußerlich stigmatisieren.

Die oben genannten Fettgewebserkrankungen zeigen im Gegensatz zu der ernährungsbedingten Lipohypertrophie (die auch eine Fett-Ablagerung im Sinne der Fettverteilungsstörung zur Folge hat) pathologisch eindeutig abzugrenzende Gewebszustände oder Entitäten, die durch histologische Vernarbungs- und Entzündungsparameter gekennzeichnet sind, aber auch durch Bindegewebsabkapselungen und durch Veränderungen in der histologischen Fettgewebsmorphologie selbst.

Ein weiterer Gegenstand der Erfindung ist die Verwendung der Zusammensetzung zur Herstellung eines Arzneimittels zur Behandlung von Cellulite/-is. Die Cellulitis ist eine Sonderform der hypertrophen Proliferation von Fettgewebe, welches sich zwischen der dermalen Fettfaszie (Kampa'sche Fettfaszie) und der Unterseite der Dermis befindet. Durch hormonelle Einflüsse kommt es zur verstärkten Wasserbindungskapazität dieser Fettzellen, die selbst wiederum durch Druckphänomene Lymphbahnstauungen im Bereich der initialen farnkrautartigen Lymphgefäßen herbeiführen. Im Verlaufe von mehreren Jahren bis Jahrzehnten bilden sich unregelmäßige, unter der während des Alterungsprozesses dünner werdenden Dermis disseminiert lokalisierte Fettknötchen, die teils schmerzhaften, teils dysästhetischen Charakter haben.

Ein weiterer Gegenstand der Erfindung ist die Verwendung der Zusammensetzung zur Herstellung eines Arzneimittels zur Behandlung von Pseudogynäkomastien und Lipomastie. Pseudogynäkomastien sind Fettansammlungen bei Männern rund um die Mamma (Brust), die zur Vergrößerung der Brust führen und vor allem ästhetisch indiziert sind. Die Lipomastie ist eine Form der Pseudogynäkomastie ohne Vergrößerung des Brustdrüsenkörpers.

Die Phospholipide, die in der hier beschriebenen pharmazeutischen Komposition enthalten sind, werden aus beliebigen tierischen oder pflanzlichen Stoffen hergestellt, insbesondere aus Hühnereiern, ölhaltigen Samen und Früchten, wie getrocknete Kokosnuss, Palmensamen, Erdnuss, Rapssamen, Sonnenblumenkerne, Leinsamen, Palm- und/oder Olivenöl. Am besten eignet sich dazu aber das Phospholipid, das aus Sojabohnen nach den Verfahren, die in den Europäischen Patenten EP 0 054 770 B1 und EP 0 054 769 B1 beschrieben sind, gewonnen wird.

Dieses Phospholipid wird hochgereinigt und enthält 15 bis 98 Gew.-%, bevorzugt 30 bis 98 Gew.-%, mehr bevorzugt 50 bis 98 Gew.-%, besonders bevorzugt 75 bis 98 Gew.-%, am meisten bevorzugt 75 bis 90 Gew.-% Phosphatidylcholin. Solche hochgereinigte Phospholipide können andere Komponenten der Phospholipide, insbesondere bis 15 Gew.-%, mehr bevorzugt bis 12 Gew.-% Phosphatidylethanolamin, bis 8 Gew.-% Phosphatidsäure, bis 10 Gew.-% Phosphatidylinositol, bis 6 Gew.-% Lysophosphatidylcholin oder Lysophosphatidylethanolamin, Spuren von Phosphatidylserin sowie andere Lipide in geringen Mengen enthalten.

Die Erfindung betrifft auch den Einsatz der Glycyrrhizinsäure, worin die Glycyrrhizinsäure als physiologisch verträgliches Salz vorliegen kann. Als Salze der Glycyrrhizinsäure werden physiologische verträgliche Salze, insbesondere Mono-, Di- oder Trinatriumsalze bzw. Kaliumsalze, Magnesium- oder Ammoniumsalze verwendet. Bevorzugt werden Mono-, Di- oder Trinatriumsalze bzw. Kaliumsalze und Ammoniumsalze und besonders bevorzugt werden Mono-, Di- oder Trinatriumsalze bzw. Kaliumsalze.

Das Massenverhältnis von Phospholipid zur Glycyrrhizinsäure beträgt 30 : 1 bis zu 0,5 : 1, bevorzugt 15 : 1 bis zu 0,5 : 1, mehr bevorzugt 4 : 1 bis zu 1 : 1, besonders bevorzugt 3 : 1 bis zu 2 : 1.

Die Phospholipid-Konzentration in der Zusammensetzung beträgt von 0,5 Gew.-% bis 30 Gew.-%, bevorzugt von 5 Gew.-% bis 25 Gew.-%.

Als empfohlener Gesamtgehalt an den Phospholipiden und Glycyrrhizinsäure oder deren Salz gilt der Gehalt von 2-80 Gew.-%. Bei diesem wird ein Gewichtsverhältnis zwischen den Phospholipiden und Glycyrrhizinsäure oder deren Salz von 3 : 1 bzw. 4 : 1 bevorzugt. Beim Gewichtsverhältnis zwischen den Phospholipiden und Glycyrrhizinsäure oder deren Salz von 2 : 1 bis 3 : 1 gilt bevorzugt der Gehalt von 2-45 Gew.-% an den Phospholipiden und Glycyrrhizinsäure oder deren Salz.

Der pH-Wert des Arzneimittels liegt im Bereich von pH 6,0 bis pH 9,0, vorzugsweise von pH 7,5 bis pH 8,5, besonders bevorzugt von pH 6,5 bis pH 7,5 und im speziellen pH 6,5 bis pH 7,0.

Gegebenenfalls werden geeignete Hilfsstoffe hinzugefügt. Als pharmakologisch zulässige Hilfsmittel dienen Zucker, insbesondere Maltose, Glukose, Milchzucker, Sorbit und Mannit, kolloidale Siliziurnsäure, Siliziumgel, Talkum, Laktose, Stärke, Gelatine, Wasser, Alkohole mit einer oder mehr Hydroxygruppen, insbesondere Ethanol, Glycerol und Propylenglycol, natürliche oder synthetische Öle insbesondere Petroleum, Mineralöl, Erdnussöl, Sojaöl, Sesamöl und Äther. Weitere geeignete Hilfsstoffe sind Cellulose, Sucrose, Malz, Reis, Fluor, Kalk, Magnesiumstearat, Natriumstearat, Glycerolmonostearat, NaCl und getrocknete Magermilch. Bevorzugte Hilfsstoffe vor allem für die subkutane Lipolyse umfassen Wasser, Alkohol, besonders Ethanol, Kochsalzlösung, Maltose und wässrige Dextrose

Die hier beschriebene pharmazeutische Zubereitung kann in trockener oder flüssiger Form verwendet werden.

Flüssige Formen umfassen Tropfen, Lösungen, Suspensionen, Emulsionen, Injektionssuspensionen oder Injektionsemulsionen und Liposomen-Mizellen-Systeme sowie Liposomen-Mizellen-Wasser-Systeme.

Bevorzugt werden flüssige Präparate wie Lösungen, Suspensionen, Emulsionen, Injektionssuspensionen oder Injektionsemulsionen. Insbesondere werden Injektionssuspensionen oder Injektionsemulsionen bevorzugt.

Insbesondere flüssige Zubereitungen wie Lösungen, Suspensionen, Emulsionen, Injektionssuspensionen oder Injektionsemulsionen aus den oben genannten Inhaltsstoffen, Hilfsstoffen oder festen Substanzen, die nach der Zugabe von Wasser, eines anderen Lösungsmittels oder eines geeigneten Puffers, wie eines Trispuffers, sofort flüssig werden, können zum Injizieren leicht verwendet werden. Bevorzugt wird ein Lyophilisat als Grundlage verwendet, um eine flüssige Zubereitung zu erzielen.

Bei Verwendung der hier beschriebenen pharmazeutischen Zubereitung als Injektion wird empfohlen, ein geeignetes Lösungsmittel zu verwenden, das keine unerwünschten Nebenwirkungen aufweist, z.B. Wasser, physiologische Kochsalzlösung, Glucose, Monohydroxy-Alkohole wie Ethanol, 2-Propanol, n-Propanol, Polyhydoxy-Alkohole wie Glyzerol und/oder Propandiol, Polyglykol wie Polyethylenglykol und/oder Miglyol, Glycerinformal, Dimethylisosorbitol, natürliche und synthetische Öle und/oder Äther, jeweils allein oder in Mischung, wobei bei Injektionen die Anwendung von Liposomen-Mizellen-Systemen empfohlen wird.

Die Restvolumina an Alkoholen nach Einengung sollten von 0 Volumenprozent (Vol. %) bis zu 20 Vol. %, bevorzugt von 0 Vol % bis 10 Vol. %, betragen.

Die am besten geeignete Anwendungsform des hier beschriebenen Präparats ist eine Mischung aus Wirkstoffen, die ein Phospholipid und die Glycyrrhizinsäure oder deren Salz in Form eines Liposomen-Mizellen-Wasser-Systems enthält. Ein solches Liposomen-Mizellen-Wasser-System, das zum Injizieren bestimmt ist, hat vorzugsweise den pH-Wert von 6,0 bis 7,5.

Die trockene Form der hier beschriebenen pharmazeutischen Zubereitung umfasst ein Lyophilisat, Tabletten, insbesondere Filmtabletten und Pillen, Pulver, Kapseln, Granulat und Dragees. Ein Lyophilisat wird bevorzugt.

Wird die Zusammensetzung in Form eines Lyophilisats hergestellt, entsteht bei der Zugabe von Wasser oder Trispuffer ein Wasser-Liposomen-System, das zum Injizieren verwendet werden kann.

Das Wasser-Liposomen-System kann auch steril filtriert werden und enthält Liposome und Mizellen in hoher Konzentration bei einer relativ geringen Größe der Teilchen von 30 nm bis 180 nm, bevorzugt 30 bis 130 nm, besonders bevorzugt 30 bis 90 nm. Diese Liposomen können unter Verwendung eines Filters mit einem Porendurchmesser von 0,2 µm steril filtriert werden.

Das hier beschriebene Präparat, das in Form einer wässrigen Lösung hergestellt wird, nimmt vorzugsweise die Form eines Liposomen-Mizellen-Systems, das hochtransparent ist, eine sehr lange Haltbarkeitsdauer hat und steril gefiltert werden kann, an. Das System kann getrocknet werden, z.B. durch Lyophilisation, wobei ein stabiles lyophilisiertes Gemisch entsteht.

Wird das hier beschriebene pharmazeutische Präparat als Injektionslösung verwendet, wird die Anwendung einer Kombination aus Wirkstoffen, welche Phospholipide und das Trinatriumsalz der Glycyrrhizinsäure enthält, wobei das Gewichtsverhältnis zwischen dem Phosphatidylcholin und dem Trinatriumsalz der Glycyrrhizinsäure 1 : 1 bis 4 : 1 und vorzugsweise 2:1 bis 3 : 1 beträgt, besonders empfohlen.

Die Herstellung der erfindungsgemäßen Zubereitungen erfolgt beispielsweise dadurch, dass man mindestens ein Phospholipid und mindestens eine Glycyrrhizinsäure im oben genannten Verhältnis zueinander in einem geeigneten Lösungsmittel löst oder dispergiert. Anschließend wird diese Lösung oder Dispersion eingeengt, und danach wird Wasser zugegeben.

Verfahren zur Herstellung der Zubereitungen werden auch in den Europäischen Patentanmeldungen EP 0 470 437 A oder EP 0 615 746 A beschrieben.

Gegebenenfalls können den erfindungsgemäß verwendeten Zubereitungen auch Antioxidationsmittel wie Ascorbinsäure, Natriumhydrogensulfit oder Natriumpyrosulfit, alpha-Tocopherol, Konservierungsmittel wie Benzylalkohol oder p-Hydroxybenzoate, oder Suspendierhilfen wie Natriumcarboxymethylcellulose zugesetzt werden.

Die Zubereitungen können gegebenenfalls auch kolloidale Strukturen wie Mizellen oder Mischmizellen enthalten. Diese Strukturen haben einen Partikeldurchmesser von 10 bis 500 Angström. Sie bestehen aus Glycyrrhizinsäure und Phospholipid. Das Massenverhältnis von Glycyrrhizinsäure zu Phospholipid beträgt in Gew.-% von 0,1 : 2 bis zu 2 : 1, bevorzugt von 1 : 2. Die Phospholipidkonzentration bei den kolloidalen Strukturen in dem Arzneimittel beträgt von 5 Gew.-% bis 15 Gew.-%, bevorzugt 10 Gew.-%. Die Herstellung der kolloidalen Strukturen erfolgt beispielsweise durch Lösung der Glycyrrhizinsäure in Wasser, wobei die Lösung etwas alkalisiert wird. Darin wird dann das Phospholipid dispergiert. Zum Abschluss wird filtriert.

Die Applikation der erfindungsgemäß eingesetzten Zubereitung und vergleichbarer Arzneiformen erfolgt durch subkutane, intraperitoneale, intramuskuläre oder intravenöse Injektion. Bevorzugt ist die subkutane Injektion.

Weiterhin wird die perkutane Applikation beansprucht, in verschiedenen Trägermedien und unter Einsatz verschiedener Hilfsmittel, beispielsweise lontophorese.

Die gleichmäßige Einbringung der erfindungsgemäß eingesetzten Zubereitungen und Arzneiformen kann auch bei bestimmten Anwendungen über ein Tumeszenzverfahren erfolgen, welches sich des hydrostatischen Drucks bedient, um eine gleichmäßige Verteilung zu gewährleisten.

Weiterhin wird die perkutane Applikation möglich, die in verschiedenen Trägermedien wie Cremes, Salben, Gele, Hydrogele, Lotionen oder Pasten erfolgen kann und unter Einsatz verschiedener Hilfsmittel, insbesondere der lontophorese, Microporation, Elektroporation oder Phonophorese.

Geeignete Zubereitungen und Arzneiformen sind beispielsweise Suspensionen, Emulsionen oder injizierbare Lösungen sowie Präparate mit protrahierter Wirkstöfffreigabe, bei deren Herstellung übliche Hilfsmittel Verwendung finden. Die Zubereitungen können auch als Konzentrat, Trockensubstanz oder Lyophilisat vorliegen, um beispielsweise die Stabilität zu erhöhen.

Vorzugsweise werden die pharmazeutischen Präparate in Dosierungseinheiten hergestellt und verabreicht, wobei jede Einheit als aktiven Bestandteil eine bestimmte Dosis der Zubereitung enthält. Bei Injektionslösungen in Ampullenform, kann diese Dosis je ml von etwa 10 mg bis zu etwa 2000 mg, bevorzugt von etwa 50 mg bis zu etwa 2000 mg, vorzugsweise von etwa 250 mg bis 500 mg bezogen auf das Phospholipid, betragen.

Für die Behandlung eines erwachsenen Patienten sind je nach Größe des zu behandelnden Fettgewebes bei der Applikation von Injektionslösungen Tagesdosen von 5 mg bis 2500 mg, bevorzugt 250 mg bis 2500 mg pro Injektionssitzung mit max. 200 Injektionen bezogen auf das Phospholipid notwendig. Die Injektionslösungen können vor Applikation auch noch verdünnt werden, vorzugsweise mit Kochsalzlösung. Unter Umständen können jedoch auch höhere oder niedrigere Tagesdosen angebracht sein. Die Dosis ist auch abhängig von der Größe der Fettansammlungen, bei kleinen Lipomen reichen Mengen von 125 mg bis 500 mg, bevorzugt 250 mg bis 500 mg pro Lipom bezogen auf das Phospholipid völlig aus. Die Verabreichung der Tagesdosis kann sowohl durch Einmalgabe in Form einer einzelnen Dosierungseinheit oder aber mehrerer kleinerer Dosierungseinheiten als auch durch Mehrfachgabe unterteilter Dosen in bestimmten Intervallen erfolgen.

Nachfolgend ist die Erfindung an Hand von Beispielen näher erläutert.

### Beispiele

### Beispiel 1: Herstellung einer Injektionslösung für die subkutane Verwendung

Eine Lösung aus:
0,2 g (8%) Trinatriumsalz der Glycyrrhizinsäure,
1,8 g (72%) Maltose und
4,5 ml Wasser
wurden mit einer Dispersion aus
0,5 g (20%) Phospholipid aus Soja und
0,5 ml (20%) 96-prozentigem Ethanol
unter Inertgasatmosphäre intensiv durchmischt.

Der Gesamtgehalt am Phospholipid und dem Salz der Glycyrrhizinsäure betrug 28 %. Das Verhältnis zwischen dem Phospholipid und dem Salz der Glycyrrhizinsäure betrug 2,5 : 1.

Die Emulsion wurde mittels Ultraschall (MSE Souiprep 150 disintegrator, England) unter 4°C für 30 Sekunden mit einminütiger Unterbrechung dispergiert. Nach ca. 10 Minuten entstand eine Liposomensuspension.

Die Liposomensuspension wurde mit einem 0,2 µm Filter 0,2 filtriert und anschließend lyophilisiert.

Die Liposomensuspension (5,0 ml) wurde innerhalb von 5 Stunden gefriergetrocknet (lyophilisiert). Dabei entstanden ca. 2,5 g lockeres, leicht gelbliches Pulver.

Für eine subkutanen Injektionslösung wurden 2,5 g des in Ampullen abgefüllten Präparats in 9,0 ml Lösungsmittel (Wasser oder Trispuffer) gelöst.

### Beispiel 2: Herstellung eines Liposom-Wasser-Systems zur subkutanen Verwendung

Es wurde eine Lösung von 50 g gereinigtem Phospholipid aus Soja (82,5 Gew.-% Phospholipid + 3,5 Gew.-% Phosphatidylchölin, bis 10 Gew.-% Phosphatidylethanolamin, 0,6 Gew.-% Lysophosphatidylcholin und maximal 10 Gew.-% sonstige Lipide) und 0,25 g Natriumsalz des Phosphatidylglyzerols in 250 ml Ethanol hergestellt. Die hergestellte Lösung wurde im Vakuum reduziert.

Die erhaltene Mischung aus Phospholipiden (ca. 20 %) wurde durch Vermischen mit 500 ml Wasser, das bereits 20,0 g (7,99 %) Trinatriumsalz der Glycyrrhizinsäure und 180,0 g (71,92 %) Isomaltose enthielt, im Inertgasstrom dispergiert.

Der Gesamtgehalt an dem Phospholipid und dem Salz der Glycyrrhizinsäure betrug 28,07 %. Das Verhältnis zwischen dem Phospholipid und dem Salz der Glycyrrhizinsäure betrug 2,5 : 1.

Diese Dispersion wurde einer Hochdruckhomogenisierung bei 600 bar fünfmal unterzogen. Das erhaltene Liposomensystem wurde mit einem Filter von 0,2 µm gefiltert und unter Inertgasatmosphäre in Ampullen von 10,0 ml abgefüllt. Das Präparat wies folgende Eigenschaften auf:
Aussehen: durchsichtige, leicht opaleszierende Flüssigkeit
pH-Wert: 6,5
Lichtdurchsichtigkeit (660 nm): 85 %.
Durchschnittsgröße der Teilchen (Laserstreuung): 75 nm.
Sterilität: den Anforderungen entsprechend
Mikroskopische Eigenschaften (Kryofixierung): 30 - 90 nm größtenteils monolamellare Liposome, einzelne bilamellare Liposome.

Die Durchsichtigkeit des in Ampullen abgefüllten Präparats wurde nach 2, 6, 9 und 12 Monaten Lagerung geprüft. Es wurden keine Unterschiede zur ursprünglichen Durchsichtigkeit des Präparats 1 festgestellt.

### Beispiel 3: Untersuchung eines membranschädigenden Effektes von Phosphatidylcholin an 3T3-L1-Zellen.

Um Effekte von Phosphatidylcholin auf die Membranintegrität und -stabilität in vitro zu testen, wurden differenzierte 3T3-L1-Zellen mit unterschiedlichen Konzentrationen an Phosphatidylcholin für 4 und 24 Stunden inkubiert und anschließend lichtmikroskopisch analysiert.

Als Modellsystem wurde die murine Präadipozyten-Zelllinie 3T3-L1 für die Untersuchungen verwendet. 3T3-L1-Zellen wurden durch einen üblichen Hormon-Cocktail (Corticosteron, Isobutylmethylxanthin, Indomethacin, Insulin) zur Adipogenese stimuliert und für weitere 8 Tage zu reifen Adipozyten differenziert.

Die reifen Adipozyten der 3T3-L1-Zellen wurden mit verschiedenen Konzentrationen an Phosphatidylcholin behandelt.

Zunächst wurde eine Phosphatidylcholin-Stammlösung hergestellt: 500 mg/ml Phosphatidylcholin gelöst in 70% Ethanol

Die verwendeten Konzentrationen an Phosphatidylcholin betrugen: 1 mg/ml, 5 mg/ml, 10 mg/ml, 15 mg/ml, 20 mg/ml

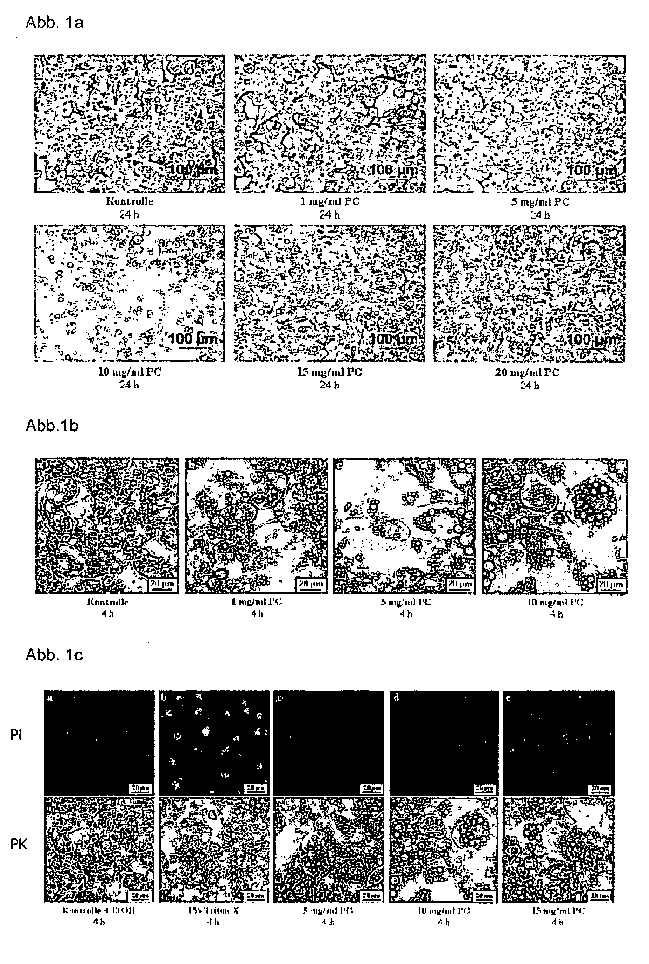

In Abbildung 1a sind lichtmikroskopische Aufnahmen der 3T3-L1-Zellen nach 24 Stunden Behandlung mit 1 mg/ml, 5 mg/ml, 10 mg/ml, 15 mg/ml und 20 mg/ml Phosphatidylcholin dargestellt, und in Abb. 1b sind die detaillierten konfokalen Laserscanning-Mikroskop (CLSM)-Aufnahmen bei einer 63-fachen Vergrößerung der mit Phosphatidylcholin behandelten 3T3-L1-Zellen zu sehen. Abb. 1c zeigt Propidiumiodid (PI) gefärbte 3T3-L1-Zellen nach Behandlung mit Phosphatidylcholin.
Abb. 1 a: Phasenkontrastaufnahmen von behandelten differenzierten 3T3-L1 Zellen in 2D-Zellkultur. Die Behandlung war wie folgt:
   (a) Kontrolle = unbehandelte Zellen in Differenzierungsmedium
   (b) 1 mg/ml Phosphatidylcholin nach 24 Stunden
   (c) 5 mg/ml Phosphatidylcholin nach 24 Stunden
   (d) 10 mg/ml Phosphatidylcholin nach 24 Stunden
   (e) 15 mg/ml Phosphatidylcholin nach 24 Stunden
   (f) 20 mg/ml Phosphatidylcholin nach 24 Stunden
      (Größenbalken = 100 µm).
Abb. 1b: Phasenkontrastaufnahmen mittels eines CLSM bei 63-facher Vergrößerung von behandelten differenzierten 3T3-L1 Zellen in 2D-Zellkultur. Die Behandlung war wie folgt:
   (a) Kontrolle = unbehandelte Zellen in Differenzierungsmedium nach 4 Stunden
   (b) 1 mg/ml Phosphatidylcholin nach 4 Stunden
   (c) 5 mg/ml Phosphatidylcholin nach 4 Stunden
   (d) 10 mg/ml Phosphatidylcholin nach 4 Stunden
      (Größenbalken = 20 µm).
Abb. 1c: Phasenkontrast-(PK)-aufnahmen mittels eines CLSM bei 63-facher Vergrößerung von behandelten differenzierten 3T3-L1 Zellen in 2D-Zellkultur nach einer PI-Färbung. Die Behandlung war wie folgt:
   (a) Kontrolle = unbehandelte Zellen in Ethanol
   (b) Positivkontrolle = unbehandelte Zellen in Differenzierungsmedium + 1% Triton
   (c) 5 mg/ml Phosphatidylcholin nach 4 Stunden
   (d) 10 mg/ml Phosphatidylcholin nach 4 Stunden
   (e) 15 mg/ml Phosphatidylcholin nach 4 Stunden

Nach der Inkubationszeit (4 Std.) wurden die Adipozyten mit 5 µg/ml PI gefärbt und unter dem CLSM analysiert. In der oberen PI-Reihe (a-e) sind die Fluoreszenzaufnahmen der PI-gefärbten Zellen gezeigt. In der unteren PK-Reihe (a-e) sind die korrespondierenden Phasenkontrastaufnahmen mit den Fluoreszenzaufnahmen übereinandergelagert (Größenbalken = 20 µm).

Die mit Phosphatidylcholin behandelten Zellen (Abb. 1 a und Abb. 1 b) zeigen keine morphologischen Unterschiede zur unbehandelten Kontrolle. Folglich hat Phosphatidylcholin keine zellschädigende Wirkung. Vergleichbare Konzentrationen (20 mg/ml) an Na-Deoxycholat riefen eine deutliche Zerstörung der Zellmembran hervor, was bei den eingesetzten Konzentrationen von Phosphatidylcholin nicht eingetreten ist.

Dass Phosphatidylcholin keine zellschädigende Wirkung hat, wurde durch die PI-Färbung der behandelten Zellen bestätigt.

Hierfür wurden differenzierte 3T3-L1 mit 5 mg/ml, 10 mg/ml und 15 mg/ml Phosphatidylcholin für 4 Stunden behandelt. Im Anschluss wurden dem Medium 5 µg/ml PI für 5 Minuten zugegeben und die Zellen unter dem CLSM analysiert (Abb. 1c).

Der Kontrolle (Abb. 1c, (a)) wurde Ethanol zugesetzt, sodass die gleiche Konzentration erzielt wurde wie in den Lösungen für die Behandlung der Zellen. Abb. 1c (a) zeigt, dass keine Zellen mit PI angefärbt sind. Daraus folgt, dass Ethanol in der verwendeten Konzentration keine zellschädigende Wirkung hat.

In Abb. 1c (b) zeigt die Positivkontrolle mit 1% Triton zahlreiche durch PI rot gefärbte Zellen. Dies weist eine Schädigung der Zellmembran nach. Hier ist die Membranstabilität und -integrität der Zelle geschädigt.

Im Vergleich dazu sind unter den behandelten Zellen (Abb. 1c (c) bis (e) keine rot gefärbten Zellen nachweisbar. Folglich weisen diese Zellen keine Schädigung der Membranstabilität und -integrität auf. Damit hat Phosphatidylcholin keinen schädigenden Effekt auf differenzierte 3T3-L1-Zellen.

Dieses Ergebnis korreliert gut mit der lichtmikroskopischen Analyse (vgl. Abb. 1a und 1b), in der ebenfalls keine zellschädigende Wirkung von Phosphatidylcholin beobachtet wurde.

### Beispiel 4: Gewinnung von "Adipose-Derived Stem Cells" (ADSC) aus humanem subkutanem Fettgewebe.

Um sich der klinischen Anwendung anzunähern, wurde in weiteren Versuchen ein anderes Modellsystem mit humanen mesenchymalen Stammzellen verwendet.

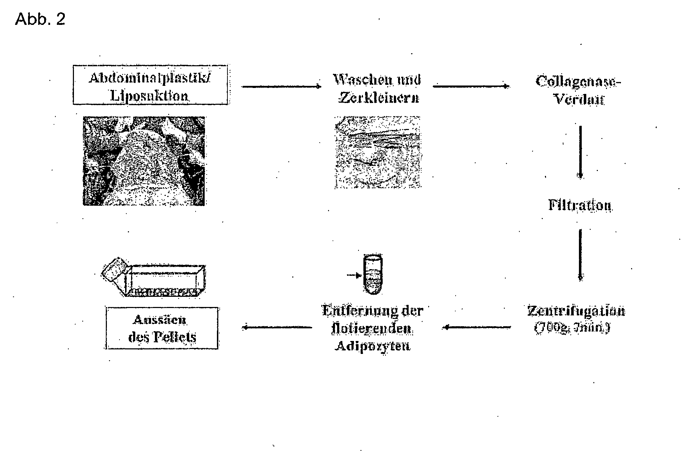

Das humane subkutane Fettgewebe kann als potentielle Quelle für adulte Stammzellen dienen. Diese sogenannten "Adipose-Derived Stem Cells" (ADSC) sind multipotent und können durch entsprechende Stimuli zu verschiedenen Zellarten, z.B. Osteoblasten, Chondrozyten, Adipozyten differenziert werden. Die Isolation von ADSCs aus humanem subkutanem Fettgewebe, das bei plastisch reduktiven Eingriffen entfernt wird, ist in Abbildung 2 schematisch gezeigt.

Zunächst wurde das Fett mit Puffer gewaschen, um hämatopoetische Zellen zu entfernen, und anschließend zerkleinert. Die entstandenen Fettgewebsstücke wurden mit Collagenase verdaut. Durch Zentrifugieren des verdauten Gewebes wurde die stromal-vaskuläre Fraktion abgetrennt und die flotierenden, reifen Adipozyten verworfen. Die stromal-vaskuläre Fraktion im Pellet setzt sich aus einer heterogenen Zellpopulation von Blutzellen, Fibroblasten, Perizyten, Endothelzellen sowie Präadipozyten zusammen.

Diese Zellpopulation wurde in eine Kulturflasche mit Medium überführt, wobei ein Teil der Zellen adhäriert. Durch Zugabe eines geeigneten Cocktails bestehend aus Insulin, Dexamethason, Indomethacin und Isobutylmethylxanthin oder bestehend aus Insulin, Cortisol, Troglitazon, Triiodothyronin und Isobutylmethylxanthin wurden die Zellen zur Adipogenese stimuliert und eine verbesserte adipogene Differenzierung bewirkt.

### Beispiel 5: Untersuchung eines membranschädigenden Effektes von Phosphatidylcholin und Na-Deoxycholat an ADSCs

### Na-Deoxycholat (Na-DC)

Die ADSCs aus Beispiel 4 wurden durch einen Hormon-Cocktail (Insulin, Cortisol, Troglitazon, Triiodothyronin und Isobutylmethylxanthin) zur Adipogenese stimuliert und anschließend weitere 21 Tage lang zu reifen Adipozyten differenziert.

Anschließend erfolgte die Behandlung mit Na-Deoxycholat. Dafür wurden folgende Konzentrationen eingesetzt:
0,01 mg/ml, 0,05 mg/ml, 0,075 mg/ml, 0,1 mg/ml und 0,5 mg/ml.
Als Kontrolle wurden unbehandelte ADSCs verwendet.
Die Inkubationszeit betrug 4 Stunden.

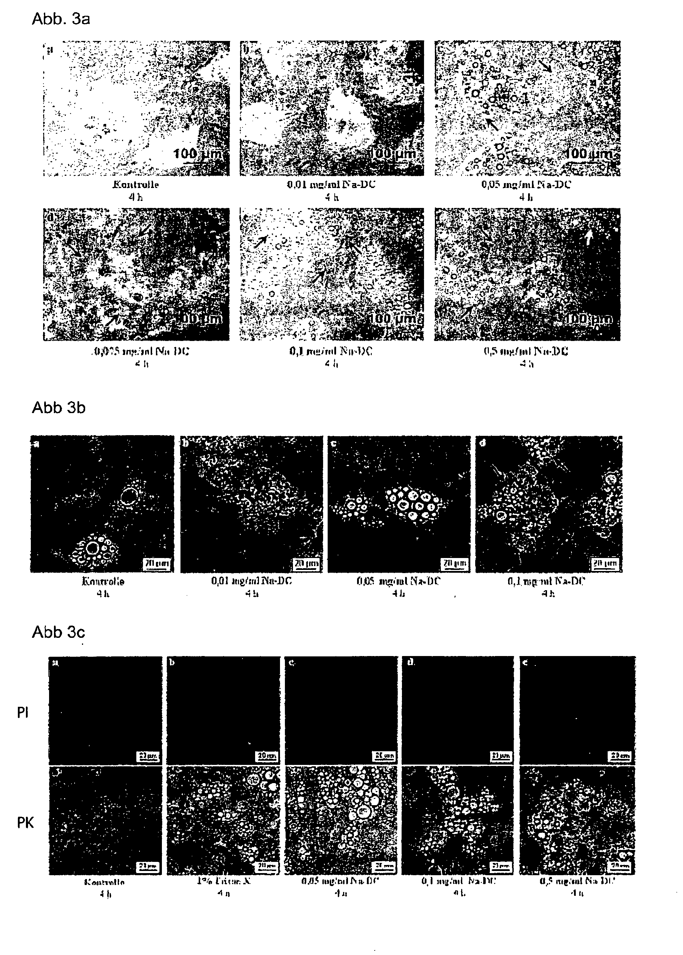

Dieser Konzentrationsbereich hat sich bei den 3T3-L1 bereits als effektiv herausgestellt. Nach der Inkubationszeit von 4 Stunden wurden die differenzierten, behandelten Zellen anschließend lichtmikroskopisch betrachtet (Abb. 3a).

Zusätzlich wurden mit Na-Deoxycholat behandelte ADSCs im CLSM untersucht. Die differenzierten, reifen Adipozyten wurden mit:
0,01 mg/ml, 0,05 mg/ml und 0,1 mg/ml Na-Deoxycholat für 4 Stunden behandelt. Als Kontrolle wurden unbehandelte ADSCs verwendet.
Anschließend erfolgte die mikroskopische Analyse im CLSM bei 63-facher Vergrößerung (Abb. 3b).

Eine weitere Analyse einer zellschädigenden Wirkung von Na-Deoxycholat auf ADSCs erfolgte durch die PI-Färbung der behandelten Zellen und anschließende Analyse unter dem CLSM. Die differenzierten, reifen Adipozyten wurden mit: 0,05 mg/ml, 0,1 mg/ml und 0,5 mg/mi Na-Deoxycholat für 4 Stunden behandelt. Als Kontrolle wurden unbehandelte ADSCs verwendet.

Anschließend wurden die Zellen in einem Medium mit 5 µg/ml Propidiumiodid für 5 Minuten inkubiert. Danach erfolgte die mikroskopische Analyse im CLSM bei 63-facher Vergrößerung (Abb. 3c)
Abb. 3a: Lichtmikroskopische Aufnahmen differenzierter ADSCs nach Behandlung mit verschiedenen Konzentrationen an Na-Deoxycholat (Na-DC) in 2D-Zellkultur. Die Behandlung war wie folgt:
   (a) Kontrolle = unbehandelte Zellen in Differenzierungsmedium 4 Stunden
   (b) 0,01 mg/ml Na-DC nach 4 Stunden
   (c) 0,05 mg/ml Na-DC nach 4 Stunden
   (d) 0,075 mg/ml Na-DC nach 4 Stunden
   (e) 0,1 mg/ml Na-DC nach 4 Stunden
   (f) 0,5 mg/ml Na-DC nach 4 Stunden
      Schwarze Pfeile deuten auf Zellen mit geschädigter Membran bzw. auf Zellfragmente. Weiße Pfeile kennzeichnen freie Lipidtropfen. (Größenbalken = 100 µm).
Abb. 3b: CLSM-Aufnahmen differenzierter ADSCs nach Behandlung mit verschiedenen Konzentrationen Na-Deoxycholat (Na-DC) in 2D-Zellkultur. Die Behandlung war wie folgt:
   (a) Kontrolle = unbehandelte Zellen in Differenzierungsmedium 4 Stunden
   (b) 0,01 mg/ml Na-DC nach 4 Stunden
   (c) 0,05 mg/ml Na-DC nach 4 Stunden
   (d) 0,1 mg/ml Na-DC nach 4 Stunden
   Schwarze Pfeile deuten auf Zellen mit geschädigter Membran bzw. auf Zellfragmente. Weiße Pfeile kennzeichnen freie Lipidtropfen.
   (Größenbalken = 20 µm).
Abb. 3c: Konfokale Laserscanning mikroskopische Aufnahmen differenzierter ADSCs nach Behandlung mit verschiedenen Konzentrationen Na-Deoxycholat (Na-DC) in 2D-Zellkultur. Die Behandlung war wie folgt:
   (a) Kontrolle = unbehandelte Zellen in Differenzierungsmedium 4 Stunden
   (b) Positivkontrolle = unbehandelte Zellen in Differenzierungsmedium + 1 % Triton
   (c) 0,05 mg/ml Na-DC nach 4 Stunden
   (d) 0,1 mg/ml Na-DC nach 4 Stunden
   (e) 0,5 mg/ml Na-DC nach 4 Stunden

Nach der Inkubationszeit (4 Std.) wurden die Adipozyten mit 5 µg/ml PI gefärbt und unter dem CLSM analysiert. In der oberen PI-Reihe (a-e) sind die Fluoreszenzaufnahmen der PI-gefärbten Zellen gezeigt. In der unteren PK-Reihe (a-e) sind die korrespondierenden Phasenkontrastaufnahmen mit den Fluoreszenzaufnahmen übereinandergelagert. (Größenbalken = 20 µm).

Eine sehr geringe Na-Deoxycholat-Konzentration von 0,01 mg/ml hatte keine Auswirkungen auf die Zellen (Abb. 3a (b)). Es waren keine Unterschiede zu der Kontrollgruppe (Abb. 3a (a)) zu erkennen. Die Adipozyten waren vital und hatten eine intakte Zellmembran. Eine Erhöhung der Konzentration ab 0,05 mg/ml rief bereits leichte Schädigungen der Zellmembran hervor (Abb. 3a (c)). Dies wurde bei den nächst höheren Konzentrationen noch verstärkt (Abb. 3a (d,e)). Die höchste Dosis von 0,5 mg/ml übte deutliche toxische Wirkung auf die Zellen aus (Abb. 3a (f)). Die Adipozyten waren tot, die Zellmembranen komplett zerstört, so dass im Wesentlichen nur noch freie Lipidtropfen und Zellfragmente vorhanden waren. Die Wirkung des Na-Deoxycholats auf die ADSCs unterschied sich nicht von der auf die 3T3-L1. Es konnten identische Dosis-Wirkungs-Beziehungen aufgestellt werden. Bei beiden Modellsystemen traten ab 0,05 mg/ml Na-Deoxycholat membranschädigende Effekte auf, und eine Konzentration von 0,5 mg/ml stellte sich als stark toxisch heraus.

Durch die genauere Betrachtung der behandelten Zellen unter dem konfokalen Mikroskop konnten die zuvor gemachten Beobachtungen bestätigt werden. Während eine geringe Konzentration von 0,01 mg/ml Na-Deoxycholat (Abb. 3b (b)) keine zellschädigende Wirkung auf die Zelle ausübte, rief eine Konzentration von 0,1 mg/ml (Abb. 3b (d)) starke Membranschäden hervor. Dies weist einen deutlichen membranschädigenden Effekt des Na-Deoxycholat nach.

Dieser Effekt wurde durch Färbung der ADSCs Zellen nach Behandlung mit Na-Deoxycholat mit Propidiumiodid erneut bestätigt.

Die Positivkontrolle mit 1% Triton zeigte deutlich durch PI rot gefärbte Zellen, was auf membrangeschädigte Zellen hinweist. (Abb. 3c (b)).

Nach einer Behandlung mit 0,05 mg/ml Na-Deoxycholat waren keine rot gefärbten Zellen und damit keine Zellen mit geschädigter Membranintegrität und - stabilität nachweisbar (Abb. 3c (c)). Nach einer Behandlung mit 0,1 mg/ml Na-Deoxycholat waren vereinzelte rot gefärbte Zellen und damit wenige Zellen mit geschädigter Membranintegrität und -stabilität nachweisbar (Abb. 3c (d)). Nach einer Behandlung mit 0,5 mg/ml Na-Deoxycholat waren viele und deutlich rot gefärbte Zellen und damit viele Zellen mit geschädigter Membranintegrität und - stabilität nachweisbar (Abb. 3c (e)).

Dies zeigt deutlich einen membranschädigenden Effekt der Na-Deoxycholat-Konzentration von 0,5 mg/ml auf die ADSCs nach.

### Phosphatidylcholin (PC)

Analog zu den Versuchen mit Na-Deoxycholat wurde für Phosphatidylcholin ebenfalls ein membranschädigender Effekt untersucht.

Hierzu wurden analog zu den Versuchen mit Na-Deoxycholat ADSCs aus Beispiel 4 verwendet und durch einen Hormon-Cocktail (Insulin, Cortisol, Troglitazon, Triiodothyronin und Isobutylmethylxanthin) zu reifen Adipozyten differenziert.

Anschließend erfolgte die Behandlung mit Phosphatidylcholin. Dafür wurden folgende Konzentrationen eingesetzt:
1 mg/ml, 5 mg/ml, 10 mg/ml, 15 mg/ml und 20 mg/ml
Als Kontrolle wurden unbehandelte ADSCs verwendet.

Die Inkubationszeit betrug 4 Stunden.

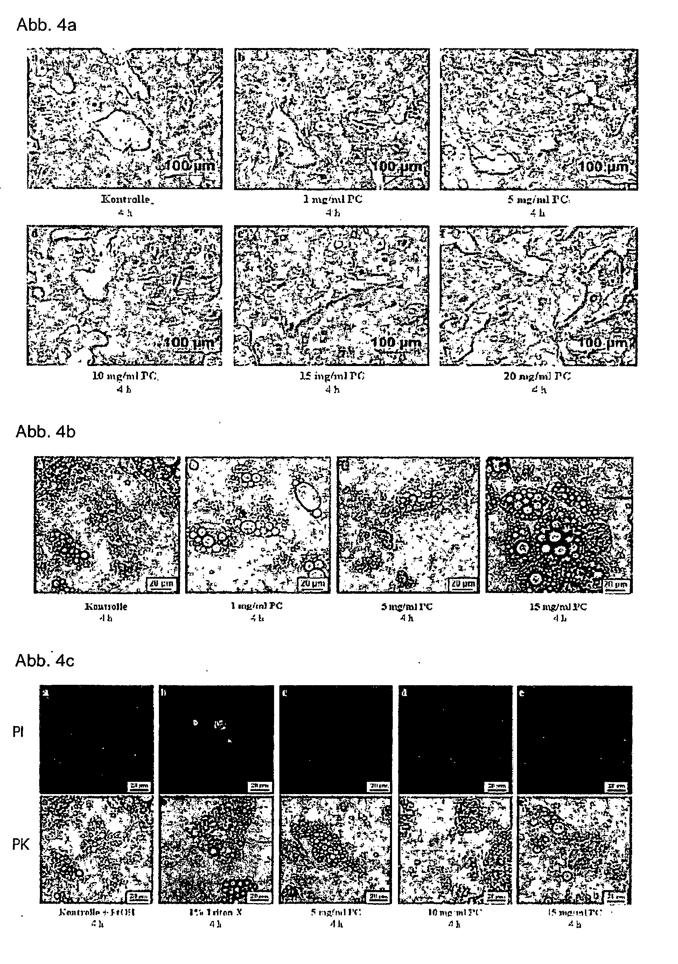

Dieser Konzentrationsbereich hat sich bei den 3T3-L1 bereits als effektiv herausgestellt. Nach der Inkubationszeit von 4 Stunden wurden die differenzierten, behandelten Zellen anschließend lichtmikroskopisch betrachtet (Abb. 4a).

Zusätzlich wurden mit Phosphatidylcholin behandelte ADSCs im CLSM untersucht. Die differenzierten, reifen Adipozyten wurden mit:
1 mg/ml, 5 mg/ml und 15 mg/ml Phosphatidylcholin für 4 Stunden behandelt. Als Kontrolle wurden unbehandelte ADSCs verwendet.

Anschließend erfolgte die mikroskopische Analyse im CLSM bei 63-facher Vergrößerung (Abb. 4b).

Eine weitere Analyse einer zellschädigenden Wirkung von Phosphatidylcholin auf ADSCs erfolgte durch die PI-Färbung der behandelten Zellen und anschließender Analyse unter dem CLSM. Die differenzierten, reifen Adipozyten wurden mit:
5 mg/ml, 10 mg/ml und 15 mg/ml Phosphatidylcholin für 4 Stunden behandelt. Als Kontrolle wurden unbehandelte ADSCs verwendet.

Anschließend wurden die Zellen in einem Medium mit 5 µg/ml Propidiümiodid für 5 Minuten inkubiert. Danach erfolgte die mikroskopische Analyse im CLSM bei 63-facher Vergrößerung (Abb. 4c/26).
Abb. 4a: Lichtmikroskopische Aufnahmen differenzierter ADSCs nach Behandlung mit verschiedenen Konzentrationen an Phosphatidylcholin (PC) in 2D-Zellkultur. Die Behandlung war wie folgt:
   (a) Kontrolle = unbehandelte Zellen in Differenzierungsmedium 4 Stunden
   (b) 1 mg/ml PC nach 4 Stunden
   (c) 5 mg/ml PC nach 4 Stunden
   (d) 10 mg/ml PCnach 4 Stunden
   (e) 15 mg/ml PC nach 4 Stunden
   (f) 20 mg/ml PC nach 4 Stunden
   (Größenbalken = 100 µm).
Abb. 4b: CLSM-Aufnahmen differenzierter ADSCs nach Behandlung mit verschiedenen Konzentrationen Phosphatidylcholin (PC) in 2D-Zellkultur. Die Behandlung war wie folgt:
   (a) Kontrolle = unbehandelte Zellen in Differenzierungsmedium 4 Stunden
   (b) 1 mg/ml PC nach 4 Stunden
   (c) 5 mg/ml PC nach 4 Stunden
   (d) 15 mg/ml PC nach 4 Stunden
   (Größenbalken = 20 µm).
Abb. 4c: CLSM-Aufnahmen differenzierter ADSCs nach Behandlung mit verschiedenen Konzentrationen Phosphatidylcholin (PC) in 2D-Zellkultur. Die Behandlung war wie folgt:
   (a) Kontrolle = unbehandelte Zellen in Differenzierungsmedium 4 Stunden
   (b) Positivkontrolle = unbehandelte Zellen in Differenzierungsmedium + 1 % Triton
   (c) 5 mg/ml PC nach 4 Stunden
   (d) 10 mg/ml PC nach 4 Stunden
   (e) 15 mg/ml PC nach 4 Stunden

Nach der Inkubationszeit (4 Std.) wurden die Adipozyten mit 5 µg/ml PI gefärbt und unter dem CLSM analysiert. In der oberen PI-Reihe (a-e) sind die Fluoreszenzaufnahmen der PI-gefärbten Zellen gezeigt. In der unteren PK-Reihe (a-e) sind die korrespondierenden Phasenkontrastaufnahmen mit den Fluoreszenzaufnahmen übereinandergelagert. (Größenbalken = 20 µm).

Entsprechend zu der Beobachtung bei 3T3-L1 (Beispiel 3) wurde in dem ADSC-Zellmodell für Phosphatidylcholin keine zellschädigende Wirkung nachgewiesen (Abb. 4a). Unabhängig von der verwendeten PC-Konzentration sind die Zellen vital, und weisen keine morphologischen Veränderungen auf.

Im Vergleich zu Na-DC wurde bereits bei eine Konzentration von 0,05 mg/ml Na-DC eine Schädigung nachgewiesen und bei einer Konzentration von 0,5 mg/ml waren die ADSCs tot (Abb. 3c).

Eine Betrachtung der ADSCs nach Behandlung mit Phosphatidylcholin im CLSM bestätigt, dass keine Schädigung der Zellen vorliegt (4b). Sowohl die unbehandelte Kontrolle wie auch die mit 15 mg/ml PC behandelten ADSCs wiesen eine intakte Morphologie auf. Folglich übt Phosphatidylcholin keinen zellschädigenden Effekt aus.

Eine PI-Färbung von mit Phosphatidylcholin behandelten ADSCs bestätigte, dass Phosphatidylcholin *in vitro* keine zellschädigenden Effekte hat.

Die Positivkontrolle mit 1% Triton zeigte deutlich durch PI rot gefärbte Zellen, was auf membrangeschädigte Zellen hinweist. (Abb. 4c (b)).

Nach einer Behandlung mit 5, 10 und 15 mg/ml Na-Deoxycholat waren nach PI-Färbung jeweils keine rot gefärbten Zellen und damit keine Zellen mit geschädigter Membranintegrität und -stabilität nachweisbar (Abb. 4c (c) bis (e)).

Dies zeigt deutlich, dass durch die Behandlung mit Phosphatidylcholin kein membranschädigender Effekt auf die ADSCs ausgeübt wurde.

Im Gegensatz dazu wurde die Membranintegrität und -stabilität der ADSCs durch Na-Deoxycholat deutlich geschädigt (Abb. 3c (d e))

Die Versuche zu Na-Deoxycholat und Phosphatidylcholin zeigen, dass nur Na-Deoxycholat eine zellschädigende Wirkung ausübt, aber nicht Phosphatidylcholin. Phosphatidylcholin ruft keine Schädigung der Zellmembranen bzw. Zellen hervor. Diese Beobachtungen wurden durch Einsatz mehrerer Methoden wie lichtmikroskopische Untersuchungen, Propidiumiodidfärbung mit anschließender Fluoreszenzmikroskopie bestätigt.

Die Wirkung der Substanzen veränderte sich im untersuchten Zeitraum über die Zeit nicht; eine Inkubation von 2 Stunden zeigte bereits die gleichen Effekte wie eine 24-stündige Inkubation. Die Versuche mit Na-Deoxycholat haben ergeben, dass dieses Gallensalz membrandestabilisierend und nekrotisch wirkt. Nach Beurteilung der *in vitro* benötigten Dosen ist es wahrscheinlich, dass auch die *in vivo* eingesetzten Mengen an Na-Deoxycholat zellschädigend wirken.

Für Phosphatidylcholin konnte *in vitro* keine zellschädigende Wirkung nachgewiesen werden, was auch *in vivo* wahrscheinlich der Fall sein wird. Dieses Phospholipid wirkte nicht zellschädigend.

### Beispiel 6: Untersuchung zur Kombination aus Phosphatidylcholin, Glycyrrhizinat und Maltose

Es wurde die Wirkung einer neuen Substanzkombination untersucht. Diese Kombination setzt sich zusammen aus
50 mg/ml Phosphatidylcholin (PC),
20 mg/ml Trinatrium Glycyrrhizinat, und
180 mg/ml Maltose.

Es wurden zunächst Dosis-Wirkungs-Beziehungen hinsichtlich membranschädigender bzw. zytotoxischer Effekte ermittelt, und auf lipolytische Aktivität untersucht.

Als Modellsystem wurde die murine Präadipozyten-Zelllinie 3T3-L1 für die Untersuchungen verwendet. 3T3-L1-Zellen wurden durch einen Hormon-Cocktail (Corticosteron, Isobutylmethylxanthin, Indomethacin, Insulin) zur Adipogenese stimuliert und für weitere 8 Tage zu reifen Adipozyten differenziert.

Die reifen Adipozyten der 3T3-L1-Zellen wurden mit verschiedenen Konzentrationen an Phosphatidylcholin zwischen 0,1 mg/ml und 50 mg/ml behandelt. Das Phosphatidylcholin wurde mit 2 - 20 mg/ml Glycyrrhizinat und 18 - 180 mg/ml Maltose kombiniert.

Die Inkubation der reifen 3T3-L1-Zellen erfolgte für eine Dauer von 4 Stunden mit der Substanzkombination.

Anschließend wurden die behandelten Zellen bei einer 63-fachen Vergrößerung lichtmikroskopisch auf einen membrandestabilisierenden und zytotoxischen Effekt hin untersucht.

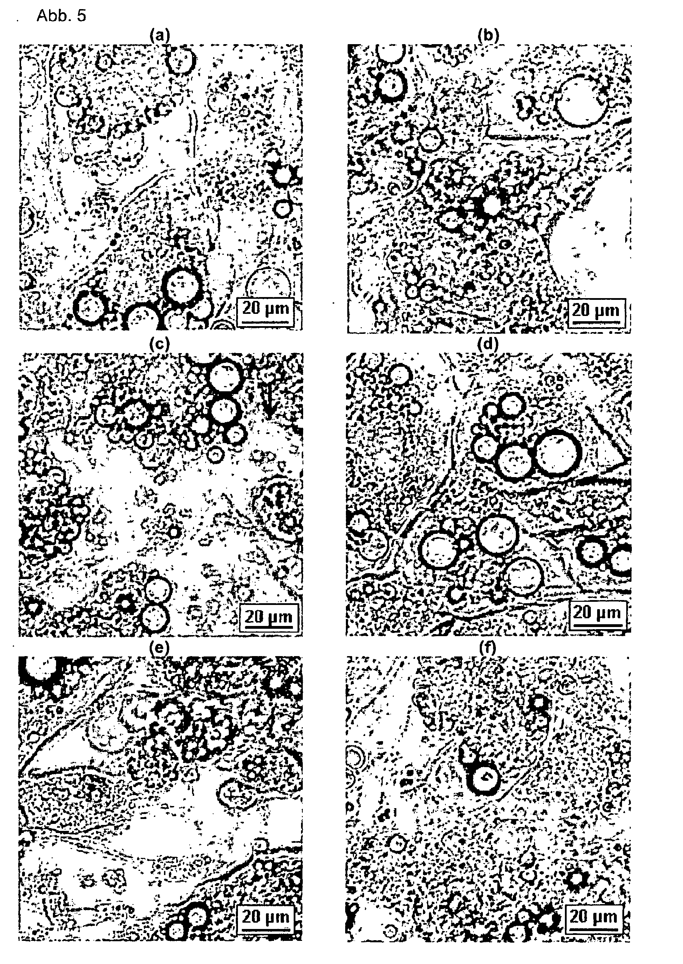

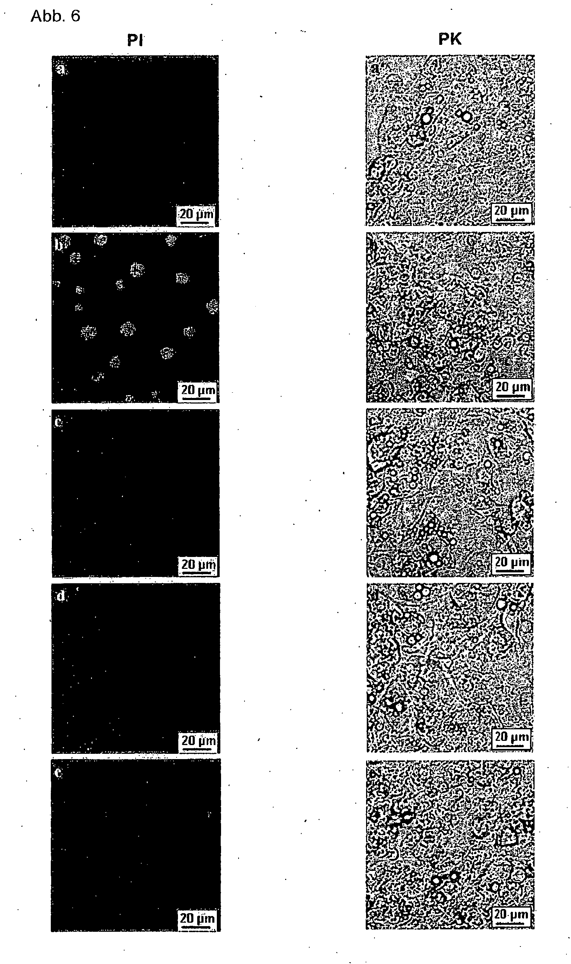

Die Ergebnisse sind in den Abbildungen 5 und 6 graphisch dargestellt:
Abb. 5: Phasenkontrast-Aufnahmen mittels eines CLSM von behandelten differenzierten 3T3-L1 Zellen in 2D-Zellkultur. Die Behandlung war wie folgt:
   (a) Kontrolle = unbehandelte Zellen in Differenzierungsmedium
   (b) 5 mg/ml PC + 2 mg/ml Glycyrrhizinat + 18 mg/ml Maltose
   (c) 10 mg/ml PC + 4 mg/ml Glycyrrhizinat + 36 mg/ml Maltose
   (d) 20 mg/ml PC + 8 mg/ml Glycyrrhizinat + 72 mg/ml Maltose
   (e) 30 mg/ml PC + 12 mg/ml Glycyrrhizinat + 108 mg/ml Maltose
   (f) 50 mg/ml PC + 20 mg/ml Glycyrrhizinat + 180 mg/ml Maltose
   (Größenbalken = 20 µm).
Abb.6: Aufnahmen mittels eines CLSM von behandelten differenzierten 3T3-L1 Zellen in 2D-Zellkultur. Die Behandlung war wie folgt:
   (a) Negativkontrolle = unbehandelte Zellen in Differenzierungsmedium
   (b) Positivkontrolle = unbehandelte Zellen in Differenzierungsmedium + 1 % Triton X
   (c) 20 mg/ml PC + 8 mg/ml Glycyrrhizinat + 72 mg/ml Maltose
   (d) 30 mg/ml PC + 12 mg/ml Glycyrrhizinat + 108 mg/ml Maltose
   (e) 50 mg/ml PC + 20 mg/ml Glycyrrhizinat + 180 mg/ml Maltose Nach der Inkubationszeit (4 Std.) wurden die Adipozyten mit 5 µg/ml PI gefärbt und unter dem CLSM analysiert. In der linken PI-Spalte (a-e) sind die Fluoreszenzaufnahmen der PI-gefärbten Zellen gezeigt. In der rechten PK-Spalte (a-e) sind die korrespondierenden Phasenkontrastaufnahmen mit den Fluoreszenzaufnahmen übereinandergelagert. (Größenbalken = 20 µm).

Die mit der Substanzkombination behandelten Adipozyten unterschieden sich nicht von den Kontrollzellen (Kontrolle Abb. 5a). Lichtmikroskopisch zeigten sie die gleiche Morphologie wie die Zellen der Kontrolle, waren vital und hatten eine intakte Zellmembran. Die Substanzkombination übte unabhängig von der Konzentration keine sichtbaren membranschädigenden Effekte auf die Zelle aus (Abb. 5).

Um die intakte Membranintegrität zu bestätigen, wurde eine PI-Färbung der behandelten 3T3-L1-Zellen und der Kontrolle durchgeführt. Dazu wurden Substanzkombinationen mit folgenden Konzentrationen verwendet:
20 mg/ml, 30 mg/ml und 50 mg/ml Phosphatidylcholin;
8 mg/ml, 12 mg/ml und 20 mg/ml Glycyrrhizinat; und
72 mg/ml, 108 mg/ml und 180 mg/ml Maltose.

Die Inkubationszeit betrug 4 Stunden. Anschließend wurden die behandelten Zellen sowie zwei Kontrollen mit 5 µg/ml PI für 5 Minuten inkubiert. Die Auswertung erfolgte mittels des CLSM (Abb. 6).

Die Behandlung der Zellen mit Triton X führte zu einer Schädigung der Membranintegrität. Dadurch konnte das Propidiumiodid in die Zellen eindringen und die membrandestabilisierte Zelle anfärben. Die Positivkontrolle (Abb. 6b) zeigte wie erwartet eine Färbung der durch Triton X permeabilisierten Zellen. Die unbehandelten Zellen der Negativkontrolle (Abb. 6a) waren nicht von PI angefärbt. Dies weist darauf hin, dass die Membran dieser Zellen intakt ist.

Die mit 20 mg/ml PC (Abb. 6c) und 30 mg/ml PC (Abb. 6d) behandelten Zellen wiesen keine Färbung durch PI auf. Folglich wiesen diese Zellen keine Schädigung der Membran und die verwendeten Substanzkombinationen keinen zellschädigenden Effekt auf (Abb. 6c, d).

Die mit 50 mg/ml PC (Abb. 6e) behandelten Zellen wiesen eine schwache rote Färbung der Zellen auf (Abb. 6e). Diese Zellen wurden hochdosiert mit der Substanzkombination behandelt. Dadurch wurde den Zellen das Medium komplett entzogen und ein Nährstoffmangel resultierte, was bei längerer Inkubation zu Zellschädigungen führt. Die schwache Anfärbung der mit 50 mg/ml PC behandelten Zellen kann folglich auf den Nährstoffmangel zurückgeführt werden und nicht auf eine Schädigung der Zellen durch Phosphatidylcholin.

Die Substanzkombination aus Phosphatidylcholin, Glycyrrhizinat und Maltose wies in den durchgeführten *in-vitro*-Experimenten keinerlei membranschädigende Wirkung auf. Dabei ist zu beachten, dass für den Nachweis einer potentiellen Membranschädigung durch die erfindungsgemäße Substanzkombinationen sehr hohe Konzentrationen verwendet wurden, die deutlich höher sind als die therapeutisch verwendeten Konzentrationen *in vivo.*

Die Beziehung zwischen einer *In-vivo-* und *In-vitro*-Wirkung wird als *In vivo*/*in vitro*-Korrelation bezeichnet. Die Ermittlung der *In vivo*/*in vitro*-Korrelation ist jedoch nicht trivial und verhält sich bei verschiedenen Bedingungen unterschiedlich. In vielen Fällen kann annäherungsweise von einer *In vivo*/*in vitro-*Korrelation um den Faktor 100 ausgegangen werden. Das bedeutet, dass Konzentrationen, die *in vitro* eingesetzt werden, in etwa um zwei Zehnerpotenzen unter den *in vivo* eingesetzten Dosen liegen, um vergleichbare Effekte zu erzielen. Auf dieser Grundlage würde man z.B. für Lipostabil^{®} N für die Injektions-Lipolyse die in Tabelle 1 aufgeführten *In-vitro*-Konzentrationen erwarten.

**Tabelle 1**

| | ***In vivo*** | **Erwartete *In-vitro-*Konzentration** (Faktor 100) [mg ml] | ***In vitro*** |
|---|---|---|---|
| | **eingesetzte Konzentration im Compound** | | **Zellschädigung hervorrufende Konzentration** |
| **Na-Deoxycholat** | 12.65 mg/ml | 0.1265 | ab 0.05 mg/ml |
| **Phosphatidylcholin** | 25 mg/ml | 0.25 | keine |
| **Na-DC, PC, aus Lipostabil** | 12.65 mg/ml Na-DC | 0.1265 | ab 0.05 mg/ml Na-DC |
| | 25 mg/ml PC | 0.25 | ab 0.1 mg/ml PC |

Im Falle des Na-Deoxycholat, sowohl als Einzelsubstanz als auch als Substanz im Lipostabil^{®}, stimmt die nach Berechnung erwartete *In-vitro*-Konzentration von der Größenordnung her mit den in den Versuchen ermittelten Konzentrationen gut überein (Tab. 1). Daraus kann man schließen, dass diese Substanz bei der Injektions-Lipolyse in der verwendeten Konzentration sehr wahrscheinlich einen zellschädigenden Effekt hat.

Folglich ist es äußerst unwahrscheinlich, dass die für eine *In vivo* -Therapie verwendeten Konzentrationen der erfindungsgemäßen Substanzkombinationen eine gleich schwere Membranschädigung der Zellen (und folgender Nekrose) hervorrufen werden wie die aus dem Stand der Technik. Gleiche Umrechnungsfaktoren können deshalb auch für die Kombination von Phosphatidylcholin und Glycyrrhizinsäure angenommen werden.

### Beispiel 7. In-vivo Untersuchungen zur Entzündungsreaktion der behandelten Zellen an der Maus

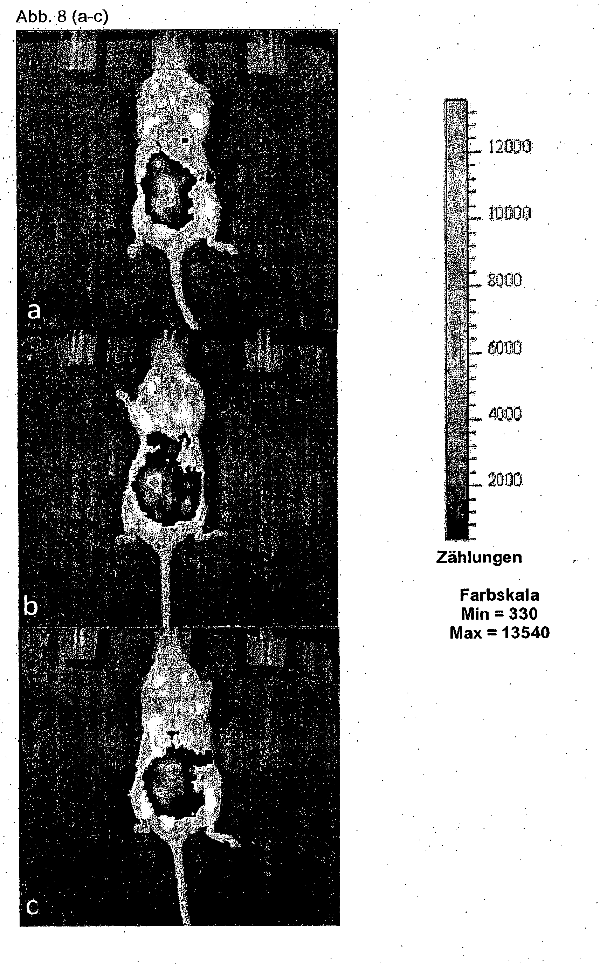

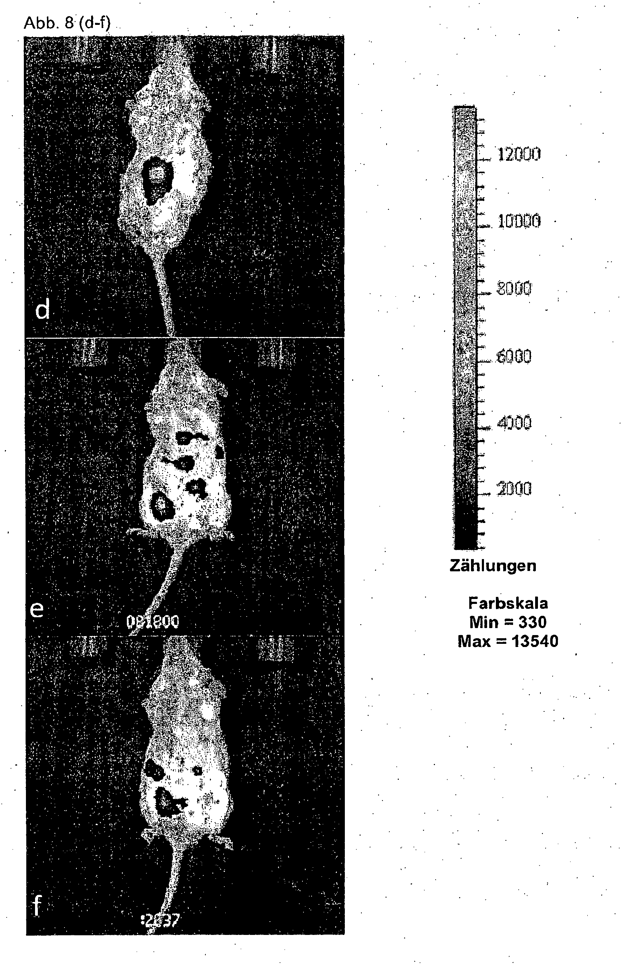

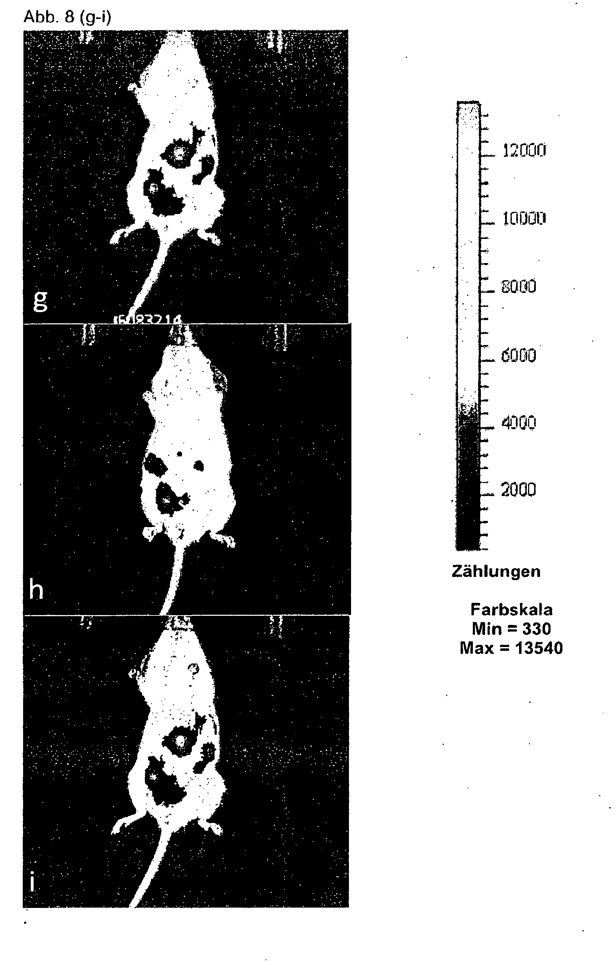

Da eine Entzündung ein Auslöser für die Migration von fettabgeleiteten Stammzellen (ADSC) ist, wurde in das rechte subkutane Fettgewebe von Balb/c Mäusen entweder eine Mischung (Abb. 8d-f) enthaltend Phosphatidylcholin (PC), Glycyrrhizinsäure (GR) und Maltose (MAL) (25 mg/ml PC, 10 mg /ml GR, 90 mg/ml MAL) PBS-Puffer (Abb. 8g-l) oder *E. coli*-Zellen (Abb. 8a-c) injiziert. Die Inkubation erfolgte für 5 Tage.

Anschließend wurde auf CD4, CD8, CD19 und CD20 hin gefärbt, um die Entzündungsreaktion der Zellen zu untersuchen.

Darüber hinaus wurden fettabgeleitete Stammzellen (ADSC) mittels eines lentiviralen Expressionsvektors eGFP/Luciferase markiert und 48 Stunden nach der Initialinjektion in das gegenüberliegende Fettgewebe der Balb/c Mäuse injiziert.

Die Migration der ADSC-Zellen wurden mittels Biolumineszenz beobachtet. Die Ergebnisse von Beispiel 7 sind in Abbildung 8 graphisch dargestellt.
Abb. 8: Biolumineszenzaufnahmen von Luciferase-markierten ADSC-Zellen nach subkutaner Injektion in das rechte Fettgewebe von Balb/C Mäusen mit:
   (a-c) *E. coli*-Zellen
   (d-f) Phosphatidylcholin + Maltose + Glycyrrhizinat (PMC)
   (g-i) PBS -Puffer

Nach 48 Stunden wurden jeweils Luciferase-markierte ADSC-Zellen intraperitoneal injiziert und die Migration der ASC-Zellen mittels Biolumineszenz beobachtet. In den Mäusen (d-i) wanderten die ASC-Zellen in die Leber und die Milz. In den Mäusen (a-c) wanderten die ASC-Zellen in die durch die *E.coli-*Injektion entzündete Region und akkumulierten dort.

In Mäusen mit PBS (Abb. 8g-l) und in Mäusen mit der Mischung enthaltend PC (Abb. 8d-f) wurde kein Unterschied in der Migration der ADSC-Zellen beobachtet. Im Gegensatz dazu wurden in Mäusen, denen *E.coli* injiziert worden war, ein Migration und Akkumulation der ADSC-Zellen in der durch die *E.coli-*Injektion ausgelöst entzündeten Region beobachtet.

Folglich wurde hier mittels Luciferase-markierten fettabgeleiteten Stammzellen (ADSC-Zellen) gezeigt, dass eine Injektion *in-vivo* einer Mischung enthaltend Phosphatidylcholin (PMC), Trinatrium-Glycyrrhizinat, und Maltose keine Entzündungsreaktion auslöst. Dies wurde daran gezeigt, dass die Luciferase-markierten ADSC-Zellen nicht in das subkutane Fettgewebe, in das die Mischung injiziert wurde, migrierten oder dort akkumulierten.

Diese Ergebnisse bestätigen, dass Phosphatidylcholin als lipolytisch aktive Substanz verwendet werden kann, ohne eine schwere Entzündungsreaktion auszulösen, wenn sie mit Glycyrrhizinsäure kombiniert wird.

### Beispiel 8. In-vitro Untersuchungen zur lipolytische Wirkung der Substanzkombination

3T3-L1-Zellen wurden durch einen Hormon-Cocktail zur Adipogenese stimuliert und für weitere 8 Tage zu reifen Adipozyten differenziert.

### Anschließend erfolgte eine 4-stündige Inkubation der Zellen mit

10 mg/ml, 25 mg/ml und 50 mg/ml Phosphatidylcholin und
4 mg/ml, 10 mg/ml und 20 mg/ml Glycyrrhizinat und
36 mg/ml, 90 mg/ml und 180 mg/ml Maltose

Die lipolytische Aktivität wurde mittels eines Lipolyse-Assays, wie nachfolgend beschrieben, bestimmt:

### Methode des Lipolyse-Assays:

Die Spaltung von Triglyceriden in Glycerol und die drei Fettsäuren bezeichnet man als Lipolyse. Dieser Vorgang wird hauptsächlich von der hormonsensitiven Lipase sowie von der Adipose-Triglycerid-Lipase katalysiert. Anhand eines Lipolyse-Assays kann man die lipolytische Aktivität von Zellen nachweisen.
Dieser Assay beruht auf der Messung des bei der Lipolyse entstehenden Glycerols, welches von den Zellen ins Medium sezerniert wird. Das Glycerol wird durch enzymatische Reaktionen zum Glycerol-1-phosphat und dann zum Dihydroxyacetonphosphat metabolisiert. Dabei entsteht Wasserstoffperoxid,
welches über eine Peroxidase-Farbreaktion photometrisch quantifiziert wird, Um Aussagen über die lipolytische Wirkung von Substanzen machen zu können,
vergleicht man sie zum einen mit der basalen Lipolyse-Aktivität der Zellen sowie mit einer stimulierten Lipolyse über den betaadrenergen Rezeptor. Solch eine Stimulation kann durch Isoproterenol hervorgerufen werden.

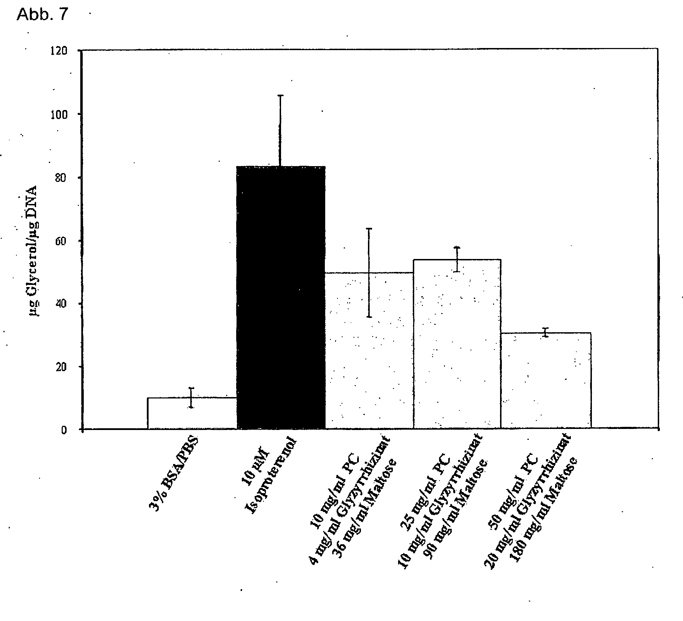

Die Ergebnisse von Beispiel 8 sind in Abb. 7 graphisch dargestellt.
Abb. 7: Graphische Darstellung der Auswertung des Lipolyse-Assays (µg Glycerol/ µg DNA) mit behandelten differenzierten 3T3-L1 Zellen. 3T3-L1 wurden durch einen hormonellen Induktionscocktail zur Adipogenese stimuliert und anschließend 8 Tage differenziert. Die Behandlung der reifen Adipozyten erfolgte jeweils für 4 Stunden in 3% BSA/PBS mit:
10 µM Isoproterenol (Positivkontrolle für eine stimulierte Lipolyse)
10 mg/ml Phosphatidylcholin + 4 mg/ml Glycyrrhizinat + 36 mg/ml Maltose
25 mg/ml Phosphatidylcholin + 10 mg/ml Glycyrrhizinat+ 90 mg/ml Maltose
50 mg/ml Phosphatidylcholin + 20 mg/ml Glycyrrhizinat + 180 mg/ml Maltose

Als Kontrolle für die basale Lipolyse-Aktivität dienten unbehandelte Zellen in 3% BSA/PBS. Die Balken geben die Standardabweichung aus n=3 an. Der Versuch wurde zweimal durchgeführt. (PC = Phosphatidylcholin)

Der erste Balken in Abbildung 7 zeigt, dass die basale Lipolyse-Aktivität von nicht behandelten Zellen in 3 % BSA/PBS bei 8 µg Glycerol/µg DNA liegt. Ausgehend von diesem Basallevel wurde die Lipolyseaktivität der weiteren Proben bestimmt. In der Positivkontrolle (Abb. 7) wurde eine Stimulation der Lipolyse durch 10 µM des β-Adrenorezeptor-Agonisten Isoproterenol induziert. Die Positivkontrolle zeigt eine lipolytische Aktivität, die auf das Achtfache des Basallevels ansteigt. Alle mit der Substanzkombination behandelten Zellen (10 mg/ml, 25 mg/ml und 50 mg/ml an PC) zeigten im Vergleich zum Basallevel eine erhöhte lipolytische Aktivität (Abb. 7). Eine Behandlung der Zellen mit der Substanzkombinätion enthaltend 10 bzw. 25 mg/ml PC bewirkte eine Erhöhung der Lipolyse-Aktivität um das 5-fache im Vergleich zum Basallevel. Die Behandlung der Zellen mit der Substanzkombination enthaltend 50 mg/ml PC führte zu einer 3-fachen Erhöhung der Lipolyse-Aktivität im Vergleich zum Basallevel (Abb. 7). Der Abfall der lipolytischen Aktivität bei der höchsten Konzentration ist wahrscheinlich, wie oben schon erklärt, darauf zurückzuführen, dass den Zellen durch Mediumentzug Nährstoffe fehlen, die sie zum Überleben bzw. zum Erhalt der normalen Funktionen, wie z.B. der lipolytischen Aktivität benötigen.

Die erfindungsgemäße Substanzkombination zeigt *in vitro* einen ausgeprägten lipolytischen Effekt.

### Vergleichsbeispiel 1: (Lipostabil^{®} enthaltend Phosphatidylcholin und Na-Deoxycholat)

Es wurde Na-Deoxycholat-Konzentrationen von 0,005 - 0,5 mg/ml (Wirkung als Einzelsubstanz ab 0,05 mg/ml) und Phosphatidylcholin-Konzentrationen von 0,01 - 1 mg/ml (keine Wirkung als Einzelsubstanz) eingesetzt.

Die Durchführung erfolgte analog zu Beispiel 8.

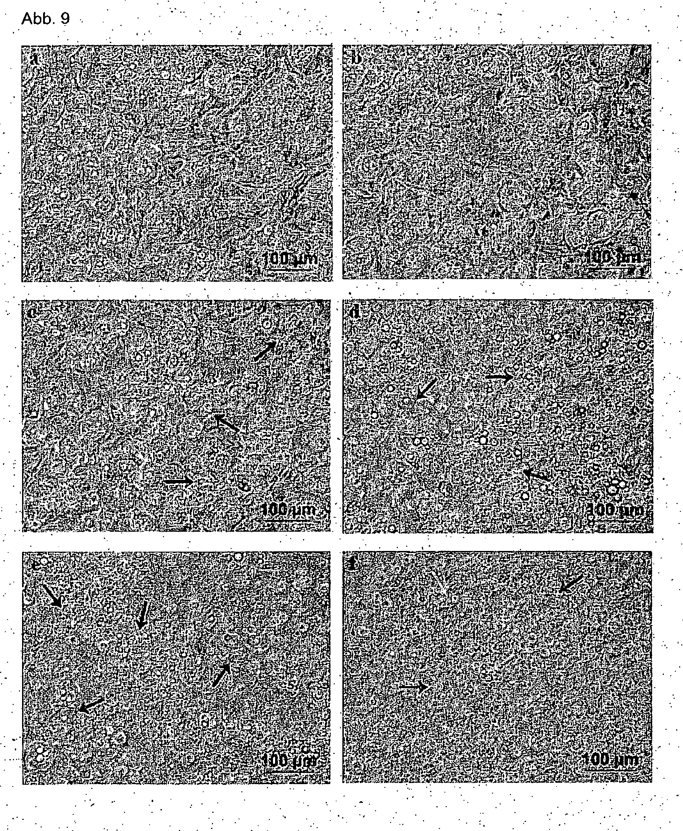

Die Ergebnisse von Vergleichsbeispiel 1 sind in Abb. 9 graphisch dargestellt.
Abb. 9: Phasenkontrastaufnahmen von behandelten differenzierten 3T3-L1-Zellen. 3T3-L1 wurden durch einen hormonellen Induktionscocktail zur Adipogenese stimuliert und anschließend 8 Tage differenziert. Die reifen Adipozyten wurden für 24 Stunden mit Phosphatidylcholin und Na-Deoxycholat aus Lipostabil^{®} mit folgenden Konzentrationen behandelt:
   (a) unbehandelte Zellen in Differenzierungsmedium
   (b) 0,01 mg/ml Phosphatidylcholin + 0,005 mg/ml Na-Deoxycholat
   (c) 0,1 mg/ml Phosphatidylcholin + 0,05 mg/ml Na-Deoxycholat
   (d) 0,25 mg/ml Phosphatidylcholin + 0,125 mg/ml Na-Deoxycholat
   (e) 0,5 mg/ml Phosphatidylcholin + 0,25 mg/ml Na-Deoxycholat
   (f) 1 mg/ml Phosphatidylcholin + 0,5 mg/ml Na-Deoxycholat

Die Analyse erfolgt mittels des Lichtmikroskops. Zellen mit geschädigter Membran bzw. Zellfragmente sind mit schwarzen Pfeilen beispielhaft gekennzeichnet, während freie Lipidtropfen durch weiße Pfeile markiert sind.

Während die geringste Konzentration an Phosphatidylcholin und Na-Deoxycholat noch keine Wirkung zeigte (Abb. 9b), war bei der nächst höheren schon eine leichte Schädigung der Zellen zu erkennen (Abb. 5c), welche mit aufsteigender Konzentration noch verstärkt wurde. In Abbildung 5e sind die Zellmembranen stark angegriffen, was zeigt, dass Lipostabil^{®} bei einer Konzentration von 0,5 mg/ml Phosphatidylcholin zusammen mit 0,25 mg/ml Na-Deoxycholat die Zellen stark schädigte und den Zelltod durch Nekrose hervorrief. Die Art der Wirkung des Lipostabils^{®} ähnelte mehr der Detergenswirkung des Na-Deoxycholats (typische Schädigung der Zellmembran), dessen Wirkung offensichtlich mehr zum Tragen kommt. Ein derart typischer membranschädigender Effekt konnte für Phosphatidylcholin als Einzelsubstanz nicht festgestellt werden, was vermuten lässt, dass im Lipostabil^{®} die zellschädigende Wirkung vorrangig von Na-Deoxycholat ausgeht. Der Vergleich des wirksamen Konzentrationsbereichs des Lipostabils^{®} mit denen der Einzelsubstanzen unterstützt die Schlussfolgerung bezüglich der vorrangigen Wirkung des Na-Deoxycholats. Während das Phosphatidylcholin als Einzelsubstanz nicht zur Wirkung kam, erzielte das Lipostabil^{®} bereits bei einer Phosphatidylcholin-Konzentration von 0,1 mg/ml leichte Effekte (bei dieser Konzentration keine Wirkung des PC als Einzelsubstanz). Dagegen kam es bei einer Einzelgabe von Na-Deoxycholat ab einer Konzentration von 0,05 mg/ml bereits zu anfänglichen Membranschädigungen, was auch beim Lipostabil^{®} bei dieser Konzentration eintrat.

### Beispiel 9: Applikation von Phosphogliv^{®} und Lipostabil^{®} bei weiblichen Probanden und Feststellung der Wirksamkeit durch subkutane Lipolyse

Das bisher bei Lebererkrankungen eingesetzte Präparat Phosphogliv^{®} i.v. enthält anstelle der DC (bei Lipostabil^{®} N i.v) Glycyrrhizinsäure. Erste experimentelle Ergebnisse und Untersuchungen an zwei Probanden deuteten auf eine gleiche Wirksamkeit des Präparates wie bei Lipostabil^{®} N i.v. bei deutlich besserer Verträglichkeit hin.

Die hierin beschriebenen Versuche weisen eine vergleichbare Wirkung von Phosphogliv^{®} zu Lipostabil^{®} bei besserer Verträglichkeit von Phosphogliv^{®} nach.

Es wurden in einer prospektiven, kontrollierten Studie sechs weibliche Probanden subkutan am linken Oberarm mit Phosphogliv^{®} und am rechten Oberarm mit Lipostabil^{®} behandelt.

Die-verwendeten Ampullen enthielten 0,5 g PPC, 0,2 g Glycyrrhizinat und 1,8 g Maltose als Lyophilisat, aufzulösen in 10 ml Wasser pro Injektion. Je nach Ausdehnung des zu injizierenden Areals erfolgten bis zu 60 subkutane Einstiche an jedem Oberarm, immer 0,5 ml im Abstand von 1,5 cm.

### Untersuchungszeitpunkte von Effekten der Präparate:

Die Behandlung dauerte insgesamt 16 Wochen. Die Untersuchungen erfolgten einen Tag vor Behandlungsbeginn (t = -1), am Tag der ersten Behandlung (t = 0),
acht Wochen nach Behandlungsbeginn (t = 8) und 16 Wochen nach Behandlungsbeginn (t = 16).

### Feststellung einer Wirkung

Zur Feststellung einer Wirkung wurde der Oberarmumfang der Probanden vermessen. Der Oberarmumfang [cm] wurde mittels eines Messschiebers (Caliper) und Maßbandes (Myo-tape) vor Behandlungsbeginn und nach acht und 16 Wochen gemessen.

Zusätzlich wurden die Blutfette Gesamtcholesterol, LDL-Cholesterol und HDL-Cholesterol in mg/dl und der atherogene Index aus LDL- zu HDL-Cholesterol bestimmt.

### Darstellung der Ergebnisse

Aus den Messwerten, den Messwertänderungen als Differenz zum Ausgangswert und der Änderung in Prozent vom Ausgangswert wurden Mittelwerte und Standardabweichung bestimmt.

**Tabelle 2: Rohdaten**

| Präparat | | | P | L | P | L | | | | | |
|---|---|---|---|---|---|---|---|---|---|---|---|
| Proband | | Zeit [t] | Cal li [cm] | Cal re [cm] | Myo li [cm] | Myo re [cm] | Gesamtcholesterol [md/dl] | LDL-Cholesterol [mg/dl] | HDL-Cholesterol [mg/dl] | Klinische Besserung* | Bemerkung |
| Nr | | | | | | | | | | | |
| 6 | I.B. | -1 | 3,1 | 3,1 | 35,0 | 35,0 | 269,0 | 169,0 | 53,8 | | |
| | I.B. | 0 | 3,1 | 3,1 | 35,0 | 35,0 | | | | | |
| | I.B. | 8 | 2,6 | 2,7 | 32,5 | 33,0 | 159,0 | 112,0 | 58,9 | Del / DelP | Ezetrol-Einnahme wahrsch. |
| | I.B. | 16 | 2,5 | 2,5 | 32,0 | 32,0 | 172,0 | 127,0 | 58,3 | Del / DelP | |
| 2 | Ch.K. | -1 | 2,9 | 2,9 | 30,5 | 30,5 | 237,0 | 136,0 | 75,4 | | |
| | Ch.K. | 0 | 2,9 | 2,9 | 30,5 | 30,5 | | | | | |
| | Ch.K. | 8 | 2,2 | 2,2 | 28,2 | 29,0 | 222,0 | 129,0 | 78,0 | Drl / DrlP | |
| | Ch.K. | 16 | 2,0 | 2,0 | 28,0 | 28,5 | 218,0 | 128,0 | 79,0 | Drl / DrlP | |
| 7 | S.A. | -1 | 3,2 | 3,3 | 31,0 | 32,0 | 212,0 | 104,0 | 88,4 | | |
| | S.A. | 0 | 3,2 | 3,3 | 31,0 | 32,0 | | | | | |
| | S.A. | 8 | 2,8 | 2,9 | 27,0 | 28,5 | 214,0 | 103,0 | 89,1 | Drl / DrlP | |
| | S.A. | 16 | 2,7 | 2,8 | 26,0 | 27,0 | | | | Drl / DrlP | |
| 1 | G.E. | -1 | 3,8 | 3,8 | 33,0 | 33,0 | 143,0 | 53,0 | 67,0 | | |
| | G.E. | 0 | 3,8 | 3,8 | 33,0 | 33,0 | | | | | |
| | G.E. | 8 | 3,2 | 3,2 | 31,8 | 31,8 | 141,0 | 51,0 | 68,0 | Del / DelP | |
| | G.E. | 16 | 2,8 | 2,8 | 30,5 | 30,5 | 142,0 | 49,0 | 54,0 | Del / DrlP | |
| 3 | M.St. | -1 | 2,9 | 3,0 | 35,0 | 36,0 | 198,0 | 120,2 | 62,8 | | |
| | M.St. | 0 | 2,9 | 3,0 | 35,0 | 36,0 | | | | | |
| | M.St. | 8 | 2,6 | 2,7 | 33,0 | 32,5 | 182,0 | 110,0 | 60,3 | Del / DelP | |
| | M.St. | 16 | 2,5 | 2,6 | 32,0 | 32,0 | 182,0 | 110,9 | 60,8 | Del / DrlP | |
| 4 | E.K. | -1 | 2,2 | 2,3 | 28,5 | 29,0 | 187,0 | 99,0 | 68,7 | | |
| | E.K. | 0 | 2,2 | 2,3 | 28,5 | 29,0 | | | | | |
| | E.K. | 8 | 1,4 | 1,5 | 26,0 | 27,0 | 177,0 | 92,0 | 69,3 | Drl / DrlP | |
| | E.K. | 16 | | | | | | | | | |

| | | | | | | | | | | | |
|---|---|---|---|---|---|---|---|---|---|---|---|
| Cal li = Caliper links; Cal re = Caliper rechts; Myo li = Myo-type links; Myo re = Myo-type rechts; P = Phosphogliv^{®}; L = Lipostabil^{®} : Del = Definite improvement of efficacy and compatibility, physician; DelP = Definite improvement of efficacy and compatibility, patient; Drl = Dramatic improvement of efficacy and compatibility, physician; DrIP = Dramatic improvement of efficacy and compatibility, patient | | | | | | | | | | | |

Der Umfang der Oberarme der Probanden unterschied sich abhängig von der gewählten Messmethode Caliper oder Myo-type. Unabhängig von der gewählten Methode konnte aber jeweils eine Abnahme des Oberarmumfangs festgestellt werden. Zwischen der Wirkung von Phosphogliv^{®} im Vergleich zu Lipostabil^{®} war kein Unterscheid erkennbar. Beide Präparate führten zu einer Reduzierung des Oberarmumfangs im gleichen Ausmaß (siehe Tabelle 3-1 und 3-2).

**Tabelle 3-1 Oberarmumfang gemessen mit Caliper [cm]**

| | | Zeit in Wochen nach Beginn der Behandlung mit | | | | | | | |
|---|---|---|---|---|---|---|---|---|---|
| | Proband | Phosphogliv^{®} (links) | | | | Lipostabil^{®} (rechts) | | | |
| | | -1 | 0 | 8 | 16 | -1 | 0 | 8 | 16 |
| | | | | | | | | | |
| 6 | I.B. | 3,1 | 3,1 | 2,6 | 2,5 | 3,1 | 3,1 | 2,7 | 2,5 |
| 2 | Ch.K. | 2,9 | 2,9 | 2,2 | 2,0 | 2,9 | 2,9 | 2,2 | 2,0 |
| 7 | S.A. | 3,2 | 3,2 | 2,8 | 2,7 | 3,3 | 3,3 | 2,9 | 2,8 |
| 1 | G.E. | 3,8 | 3,8 | 3,2 | 2,8 | 3,8 | 3,8 | 3,2 | 2,8 |
| 3 | M.St. | 2,9 | 2,9 | 2,6 | 2,5 | 3,0 | 3,0 | 2,7 | 2,6 |
| 4 | E.K. | 2,2 | 2,2 | 1,4 | | 2,3 | 2,3 | 1,5 | |
| | | | | | | | | | |
| Mittelwert | | 3,02 | 3,02 | 2,47 | 2,50 | 3,07 | 3,07 | 2,53 | 2,54 |
| Standardabweichung | | 0,52 | 0,52 | 0,62 | 0,31 | 0,49 | 0,49 | 0,60 | 0,33 |
| N | | 6 | 6 | 6 | 5 | 6 | 6 | 6 | 5 |

**Tabelle 3-2 Änderung des Oberarmumfangs mit Caliper in cm als absolute und relative Differenz zur Woche 0**

| | | Zeit in Wochen nach Beginn der Behandlung mit | | | | | | | |
|---|---|---|---|---|---|---|---|---|---|
| Proband | | Phosphogliv^{®} (links) | | | | Lipostabil^{®} (rechts) | | | |
| | | 8 | | 16 | | 8 | | 16 | |
| | | cm | % | cm | % | cm | % | cm | % |
| | | | | | | | | | |
| 6 | I.B. | -0,5 | -16,1 | -0,6 | -19,4 | -0,4 | -12,9 | -0,6 | -19,4 |
| 2 | Ch.K. | -0,7 | -24,1 | -0,9 | -31,0 | -0,7 | -24,1 | -0,9 | -31,0 |
| 7 | S.A. | -0,4 | -12,5 | -0,5 | -15,6 | -0,4 | -12,1 | -0,5 | -15,2 |
| 1 | G.E. | -0,6 | -15,8 | -1,0 | -26,3 | -0,6 | -15,8 | -1,0 | -26,3 |
| 3 | M.St. | -0,3 | -10,3 | -0,4 | -13,8 | -0,3 | -10,0 | -0,4 | -13,3 |
| 4 | E.K. | -0,8 | -36,4 | | | -0,8 | -34,8 | | |
| | | | | | | | | | |
| Mittelwert | | -0,55 | -19,21 | -0,68 | -21,22 | -0,53 | -18,29 | -0,68 | -21,04 |
| Standardabweichung | | 0,19 | 9,63 | 0,26 | 7,28 | 0,20 | 9,47 | 0,26 | 7,49 |
| N | | 6 | 6 | 5 | 5 | 6 | 6 | 5 | 5 |

Aus Tabelle 3-2 wird ersichtlich, dass durch Phosphogliv^{®} und Lipostabil^{®} mittels Caliper nach 8 Wochen eine Reduzierung des Oberarmumfangs von 19,21 % bzw. 18,29 % erfolgte. Nach 16 Wochen wurde eine Reduzierung des Oberarmumfangs von 21,22 % bzw. 21,04 % mittels Caliper festgestellt. Folglich haben beide Präparate Phosphogliv^{®} und Lipostabil^{®} die gleiche Wirksamkeit.

**Tabelle 4-1 Oberarmumfang gemessen mit Myo-tape [cm]**

| | | Zeit in Wochen nach Beginn der Behandlung mit | | | | | | | |
|---|---|---|---|---|---|---|---|---|---|
| Proband | | Phosphogliv^{®} (links) | | | | Lipostabil^{®} (rechts) | | | |
| | | -1 | 0 | 8 | 16 | -1 | 0 | 8 | 16 |
| | | | | | | | | | |
| 6 | I.B. | 35,0 | 35,0 | 32,5 | 32,0 | 35,0 | 35,0 | 33,0 | 32,0 |
| 2 | Ch.K. | 30,5 | 30,5 | 28,2 | 28,0 | 30,5 | 30,5 | 29,0 | 28,5 |
| 7 | S.A. | 31,0 | 31,0 | 27,0 | 26,0 | 32,0 | 32,0 | 28,5 | 27,0 |
| 1 | G.E. | 33,0 | 33,0 | 31,8 | 30,5 | 33,0 | 33,0 | 31,8 | 30,5 |
| 3 | M.St. | 35,0 | 35,0 | 33,0 | 32,0 | 36,0 | 36,0 | 32,5 | 32,0 |
| 4 | E.K. | 28,5 | 28,5 | 26,0 | | 29,0 | 29,0 | 27,0 | |
| | | | | | | | | | |
| Mittelwert | | 32,17 | 32,17 | 29,75 | 29,70 | 32,58 | 32,58 | 30,30 | 30,00 |
| Standardabweichung | | 2,62 | 2,62 | 3,04 | 2,64 | 2,65 | 2,65 | 2,46 | 2,21 |
| N | | 6 | 6 | 6 | 5 | 6 | 6 | 6 | 5 |

**Tabelle 4-1 Änderung des Oberarmumfangs mit Myo-tape in cm als absolute und relative Differenz zur Woche 0**

| | | Zeit in Wochen nach Beginn der Behandlung mit | | | | | | | |
|---|---|---|---|---|---|---|---|---|---|
| Proband | | Phosphogliv^{®} (links) | | | | Lipostabil^{®} (rechts) | | | |
| | | 8 | | 16 | | 8 | | 16 | |
| | | cm | % | cm | % | cm | % | cm | % |
| | | | | | | | | | |
| 6 | I.B. | -2,5 | -7,1 | -3,0 | -8,6 | -2,0 | -5,7 | -3,0 | -8,6 |
| 2 | Ch.K. | -2,3 | -7,5 | -2,5 | -8,2 | -1,5 | -4,9 | -2,0 | -6,6 |
| 7 | S.A. | -4,0 | -12,9 | -5,0 | -16,1 | -3,5 | -10,9 | -5,0 | -15,6 |
| 1 | G.E. | -1,2 | -3,6 | -2,5 | -7,6 | -1,2 | -3,6 | -2,5 | -7,6 |
| 3 | M.St. | -2,0 | -5,7 | -3,0 | -8,6 | -3,5 | -9,7 | -4,0 | -11,1 |
| 4 | E.K. | -2,5 | -8,8 | | | -2,0 | -6,9 | | |
| | | | | | | | | | |
| Mittelwert | | -2,42 | -7,62 | -3,20 | -9,81 | -2,28 | -6,97 | -3,30 | -9,89 |
| Standardabweichung | | 0,92 | 3,13 | 1,04 | 3,56 | 0,99 | 2,84 | 1,20 | 3,63 |
| N | | 6 | 6 | 5 | 5 | 6 | 6 | 5 | 5 |

Aus Tabelle 4-2 wird ersichtlich, dass durch Phosphogliv^{®} und Lipostabil^{®} gemäß der Methode Myo-tape nach 8 Wochen eine Reduzierung des Oberarmumfangs von 7,62 % bzw. 6,97 % erfolgte. Nach 16 Wochen wurde eine Reduzierung des Oberarmumfangs von 9,81 % bzw. 9,89 % mittels Myo-tape festgestellt. Folglich haben beide Präparate Phosphogliv^{®} und Lipostabil^{®} die gleiche Wirksamkeit.

Auch wenn die beiden Methoden Caliper und Myo-tape unterschiedliche Reduzierungen der Präparate nachweisen, so ist das Ausmaß der Wirksamkeit bei den beiden Präparaten Lipostabil^{®} und Phosphogliv^{®} gleichwertig.

### Beispiel 10: Feststellung von Nebenwirkungen durch die Behandlung mit Lipostabil^{®} und Phosphogliv^{®}.

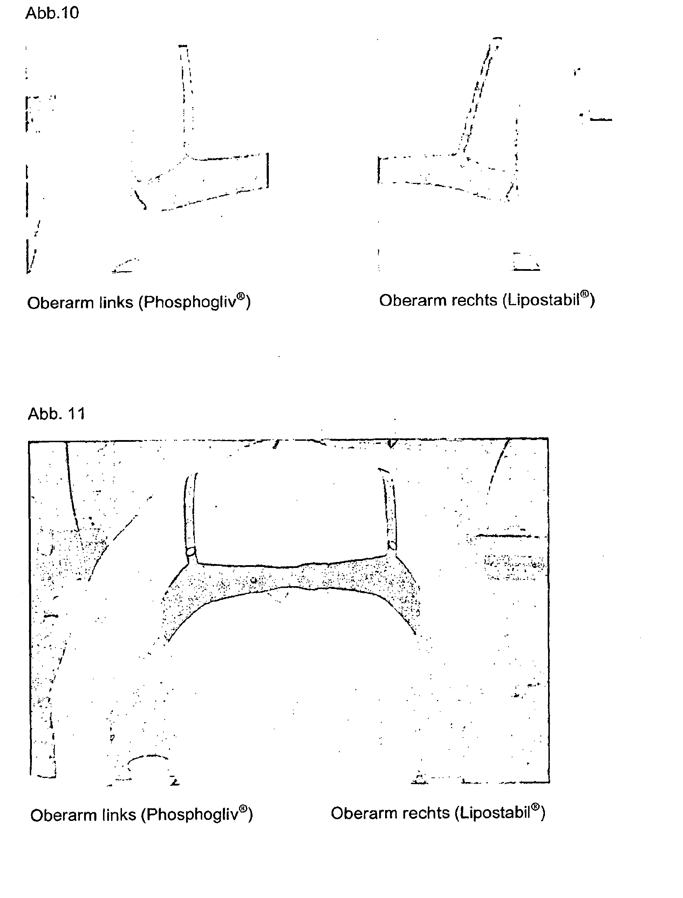

Hierzu wurden die Probandinnen wie in Beispiel 9 beschrieben behandelt und die Wirksamkeit untersucht. Um die Nebenwirkungen zu dokumentieren, wurden Aufnahmen vor und nach der Behandlung mit Phosphogliv^{®} und Lipostabil^{®} der Oberarme der Probandinnen gemacht (Abb. 10 und 11).

Nach 3 Minuten wurde bei Probandin Nr. 01 am rechten Oberarm, der mit Lipostabil^{®} behandelt wurde, eine deutliche Rötung und Schwellung festgestellt. Im Vergleich dazu wurde am linken Oberarm, der mit Phosphogliv^{®} behandelt wurde, nur eine leichte Rötung und Schwellung festgestellt. Dieser Phänotyp bestätigt die Ergebnisse der *in-vitro* Versuche zu Phosphatidylcholin, das keine membranschädigende Wirkung auf die Zellen hat (Beispiel 5, Abb. 4a-c)). Die Aufnahmen von Probandin Nr. 02 in Abb. 11 zeigen die gleiche Unterscheidung zwischen dem linken (Phosphogliv^{®}) und rechten (Lipostabil^{®}) Oberarm.

Abb. 10: Photographische Dokumentation nach Behandlung des linken (Phosphogliv^{®}) und rechten (Lipostabil^{®}) Oberarmes der Probandin Nr. 01. Die Aufnahmen wurden 3 Minuten nach der Applikation der Präparate Lipostabil^{®} und Phosphogliv^{®} gemacht.

Abb. 11: Photographische Dokumentation nach Behandlung des linken (Phosphogliv^{®}) und rechten (Lipostabil^{®}) Oberarmes der Probandin Nr. 02. Die Aufnahmen wurden 3 Minuten nach der Applikation der Präparate Lipostabil^{®} und Phosphogliv^{®} gemacht

Desweiteren wurden die Nebenwirkungen in der nachfolgenden Tabelle 5 zusammengefasst.

**Tabelle 5 Nebenwirkungen infolge der Behandlung mit Phosphogliv^{®}- und Lipostabil^{®}**

| | **t = 0** | | **t = 8** | | **t = 16** | |
|---|---|---|---|---|---|---|
| **Prob** | **P** | **L** | **P** | **L** | **P** | **L** |
| **Nr.1** | | | Nur kurzer Schmerz, nahezu keine Schwellung | Signifikante Schwellung und Schmerz, bis zu 3 Tage | Nahezu kein Schmerz | Schwellung und Rötung bis zum 4. Tag. |
| | | | | | | |
| **Nr.2** | | | Viel weniger Schwellung und Rötung | Viel mehr Schwellung und Rötung | Nahezu kein Schmerz | Schmerzen für eine Woche |
| | | | | | | |
| **Nr.3** | Nichts | Brennen und Jucken für 5 min; leichte Schmerzen für 2 Wochen | "ein Kinderspiel" überhaupt keine Schmerzen | Leichter Schmerz für 2 Wochen | Keine unerwartete Reaktion, viel weniger Beschwerden | Keine unerwartete Reaktion, viel mehr Beschwerden |
| **Nr.4** | | | Nur für etwa 3 Minuten Schmerzen an der Injektionsstelle; Schwellung und Rötung für 3 Tage; danach keine Schmerzen oder Schwellung mehr | Viel mehr Schmerz, Schwellung für 3 Tage etwa wie bei Phosphogliv. Schwellung und Rötung für 12 Tage. Sensibilität über nahezu die ganzen 8 Wochen | | |
| | | | | | | |
| **Nr. 6** | | | Nahezu keine Schmerzen | | Keine Beschwerden | Schwellung und Rötung für 3 Tage |
| | | | | | | |
| **Nr.7** | | | Nahezu keine Schmerzen | | Schmerzen und Schwellung an beiden Oberarmen leicht ertragbar. | |

| | | | | | | |
|---|---|---|---|---|---|---|
| P = Phosphogliv^{®}; L = Lipostabil^{®} | | | | | | |

Arzt und Proband sprachen nach der ersten und zweiten Behandlung in allen Fällen einheitlich sowohl von deutlicher bzw. dramatischer Verbesserung (Reduktion) der Oberarmfettpolster unter beiden Präparaten wie von deutlich besserer Verträglichkeit unter Phosphogliv^{®}. Die Hautfestigkeit war in allen Fällen nach Behandlung gut.

Die Daten aus Tabelle 5 zeigen, dass die Beschwerden der Probandinnen nach einer Behandlung mit Lipostabil^{®} mit Schwellungen, Rötungen und Schmerzen an den Injektionsstellen einhergehen. Solche Nebenwirkungen traten bei einer Behandlung mit Phosohogliv^{®} kaum bis gar nicht auf. Damit ist Phosphogliv^{®} bei den befragten Probandinnen wesentlich besser verträglich gewesen als das Präparat Lipostabil^{®}. Wie zuvor schon erwähnt, ist die bessere Verträglichkeit auf die reduzierte bzw. nicht vorkommende Zellschädigung zurückzuführen. Wie in Beispiel 5 gezeigt, schädigt Phosphatidylcholin die Zellmembran nicht, während das Na-Deoxycholat aus Lipostabil^{®} die Zellen schädigt.

### Beispiel 11: Bestimmung der Cholesterolwerte vor und nach subkutaner Lipolyse

Hierzu wurden die Bluttfette der Probandinnen über den Therapiezeitraum beobachtet. Es wurden Gesamcholesterol, LDL-(Low-Density-Lipoprotein)-Cholesterol und HDL-(High-Density Lipoprotein)-Cholesterol bestimmt. Tabelle 6 fasst die Werte zum Zeitpunkt t = -1, t = 8 Wochen und t = 16 Wochen zusammen.

Die Blutfette veränderten sich im Mittel über den Therapiezeitraum gleichgerichtet. Einzig bei den zwei Probandinnen (I.B. und Ch.K.) mit deutlich erhöhtem Gesamtcholesterol und LDL-Cholesterol fielen die Werte ab, während das HDL-Cholesterol anstieg (Tab.6). Eine Zusammenfassung der Studie ist in Tabelle 7 dargestellt.

Die weiteren biochemischen Kontrollvariablen AST, ALT, y-GT, Bilirubin, Glukose und Kreatinin blieben mit Ausnahme eines leichten erhöhten y-GT-Wertes bei Probandin S.A. vor Therapiebeginn im Normbereich.

**Tabelle 6 Chofesterolwerte [mg/dl]**

| | | Zeit in Wochen nach Beginn der Behandlung | | | | | | | | |
|---|---|---|---|---|---|---|---|---|---|---|
| Proband | | Gesamtcholesterol | | | LDL-Cholesterol | | | HDL-Cholesterol | | |
| | | -1 | 8 | 16 | -1 | 8 | 16 | -1 | 8 | 16 |
| | | | | | | | | | | |
| 6 | I.B. | 269,0 | 159,0 | 172,0 | 169,0 | 112,0 | 127,0 | 53,8 | 58,9 | 58,3 |
| 2 | Ch.K. | 237,0 | 222,0 | 218,0 | 136,0 | 129,0 | 128,0 | 75,4 | 78,0 | 79,0 |
| 7 | S.A. | 212,0 | 214,0 | | 104,0 | 103,0 | | 88,4 | 89,1 | |
| 1 | G.E. | 143,0 | 141,0 | 142,0 | 53,0 | 51,0 | 49,0 | 67,0 | 68,0 | 54,0 |
| 3 | M.St. | 198,0 | 182,0 | 182,0 | 120,2 | 110,0 | 110,9 | 62,8 | 60,3 | 60,8 |
| 4 | E.K. | 187,0 | 177,0 | | 99,0 | 92,0 | | 68,7 | 69,3 | |
| | | | | | | | | | | |
| Mittelwert | | 207,67 | 182,50 | 178,50 | 113,53 | 99,50 | 103,73 | 69,35 | 70,60 | 63,03 |
| Standardabweichung | | 43,23 | 31,17 | 31,34 | 38,97 | 26,67 | 37,32 | 11,75 | 11,39 | 11,01 |
| N | | 6 | 6 | 4 | 6 | 6 | 4 | 6 | 6 | 4 |

| | | | | | | | | | | |
|---|---|---|---|---|---|---|---|---|---|---|
| LDL/HDL-Quotient -1 Woche 1,64 (Zielwert < 3) 8 Wochen 1,41 16 Wochen 1,65 LDL-Cholesterol = Low-Density-Lipoprotein-Cholesterol HDL-Cholesterol = High-Density-Lipoprotein-Cholesterol | | | | | | | | | | |

**Tabelle 7 Zusammenfassung der Ergebnisse der Studie mit den Probanden**

| | | | Caliper [cm] | | Myo-type | | Klinische Besserung | Bemerkung |
|---|---|---|---|---|---|---|---|---|
| | | | Links | Rechts | Links | Rechts | | |
| Proband | | Zeit | Phosphogliv | Lipostabil | Phosphogliv | Lipostabil | | |
| 6 | I.B. | Screeninq | 3,1 | 3,1 | 35 | 35 | | * Ezetrol-Einnahme wahrscheinlich |
| | | 0 | 3,1 | 3,1 | 35 | 35 | | |
| | | 8 | 2,6 | 2,7 | 32,5 | 33 | Del / DelP | Gesamtcholesterol von 269,0 mg/dl auf 159,0 mg/dl |
| | | | | | | | | LDL-Cholesterol von 169,0 mg/dl auf 112,0 mg/dl |
| | | | | | | | | HDL-Cholesterol von 53,8 mg/dl auf 58,9 mg/dl |
| | | 16 | 2,5 | 2,5 | 32 | 32 | Del / DelP | Gesamtcholesterol von 159,0 mg/dl auf 172,0 mg/dl |
| | | | | | | | | LDL-Cholesterol von 112,0 mg/dl auf 127,0 mg/dl |
| | | | | | | | | HDL-Cholesterol von 58,9 mg/dl auf 58,3 |
| | | | | | | | | |
| | | | | | | | | Links keine Beschwerden, rechts bis zu 3 Tagen Schmerzen Hautfestigkeit von mäßig auf gut gebessert |
| | | | | | | | | |
| 2 | Ch.K. | Screening | 2,9 | 2,9 | 30,5 | 30,5 | | |
| | | 0 | 2,9 | 2,9 | 30,5 | 30,5 | | |
| | | 8 | 2,2 | 2,2 | 28,2 | 29 | Drl / DrlP | Gesamtcholesterol von 237,0 mg/dl auf 222,0 mg/dl |
| | | | | | | | | LDL-Cholesterol von 136,0 mg/dl auf 129,0 mg/dl |
| | | | | | | | | HDL-Cholesterol von 75,4 mg/dl auf 78,0 mg/dl |
| | | 16 | 2,0 | 2,0 | 28 | 28,5 | Drl / DrlP | Gesamtcholesterol von 222 mg/dl auf 218 mg/dl |
| | | | | | | | | LDL-Cholesterol von 129,0 mg/dl auf 128,0 mg/dl |
| | | | | | | | | HDL-Cholesterol von 78,0 mg/dl auf 79,0 mg/dl |
| | | | | | | | | |
| | | | | | | | | Links nahezu keine Schmerzen, rechts für etwa 1 Woche Hautfestigkeit von Beginn an gut |
| | | | | | | | | |
| 3 | M. St. | Screening | 2,9 | 3,0 | 35 | 36 | | |
| | | 0 | 2,9 | 3,0 | 35 | 36 | | |
| | | 8 | 2,6 | 2,7 | 33 | 32,5 | Del / DelP | Gesamtcholesterol von 198,0 mg/dl auf 182,0 mg/dl |
| | | | | | | | | LDL-Cholesterol von 120,2 mg/dl auf 110,0 mg/dl |
| | | | | | | | | HDL-Cholesterol von 62,8 mg/dl auf 60,3 mg/dl |
| | | 16 | 2,5 | 2,6 | 32 | 32 | Del / DelP | Gesamtcholesterol von 182,0 mg/dl auf 182,0 mg/dl |
| | | | | | | | | LDL-Cholesterol von 110,0 mg/dl auf 110,9 mg/dl |
| | | | | | | | | HDL-Cholesterol von 60,3 mg/dl auf 60,8 mg/dl |
| | | | | | | | | |
| | | | | | | | | Hautfestigkeit von mäßig auf gut gebessert |
| | | | | | | | | |
| 4 | E.K. | Screening | 2,2 | 2,3 | 28,5 | 29 | | |
| | | 0 | 2,2 | 2,3 | 28,5 | 29 | | |
| | | 8 | 1,4 | 1,5 | 26 | 27 | Drl / DrlP | Gesamtcholesterol von 187,0 mg/dl auf 177,0 mg/dl |
| | | | | | | | | LDL-Cholesterol von 99,0 mg/dl auf 92,0 mg/dl |
| | | | | | | | | HDL-Cholesterol von 68,7 mg/dl auf 69,3 mg/dl |
| | | | | | | | | |
| | | | | | | | | Hautfestigkeit von Beginn an gut |
| | | | | | | | | |
| 7 | S.A. | Screening | 3,2 | 3,3 | 31 | 32 | | |
| | | 0 | 3,2 | 3,3 | 31 | 32 | | |
| | | 8 | 2,8 | 2,9 | 27 | 28,5 | Drl / DrlP | Gesamtcholesterol von 212,0 mg/dl auf 214,0 mg/dl |
| | | | | | | | | LDL-Cholesterol von 104,0 mg/dl auf 103,0 mg/dl |
| | | | | | | | | HDL-Cholesterol von 88,4 mg/dl auf 89,1 mg/dl |
| | | 16 | 2,7 | 2,8 | 26 | 27 | Drl / DrlP | |
| | | | | | | | | y-GT normalisiert |
| | | | | | | | | Hautfestigkeit von Beginn an gut |
| | | | | | | | | |
| 1 | G.E. | Screening | 3,8 | 3,8 | 33 | 33 | | |
| | | 0 | 3,8 | 3,8 | 33 | 33 | | |
| | | 8 | 3,2 | 3,2 | 31,8 | 31,8 | Del / DelP | Gesamtcholesterol von 143,0 mg/dl auf 141,0 mg/dl |
| | | | | | | | | LDL-Cholesterol von 53,0 mg/dl auf 51,0 mg/dl |
| | | | | | | | | HDL-Cholesterol von 67,0 mg/dl auf 68,0 mg/dl |
| | | 16 | 2,8 | 2,8 | 30,5 | 30,5 | Del/DelP | Gesamtcholesterol von 141,0 mg/dl auf 142,0 mg/dl |
| | | | | | | | | LDL-Cholesterol von 51,0 mg/dl auf 49,0 mg/dl |
| | | | | | | | | HDL-Cholesterol von 68,0 mg/dl auf 54,0 mg/dl |
| | | | | | | | | |
| | | | | | | | | Hautfestigkeit von Beginn an gut |

| | | | | | | | | |
|---|---|---|---|---|---|---|---|---|
| Del = Definite improvement of efficacy and compatibility, physician; DelP = Definite improvement of efficacy and compatibility, patient; Drl = Dramatic improvement of efficacy and compatibility, physician; DrIP = Dramatic improvement of efficacy and compatibility, patient | | | | | | | | |

## Patentansprüche

1. Verwendung einer Zusammensetzung, enthaltend
a) mindestens ein Phospholipid;
b) Glycyrrhizinsäure oder
ein Salz der Glycyrrhizinsäure und
c) ggf. Hilfsstoffe
wobei
- der Gesamtgehalt an den Phospholipiden und der Glycyrrhizinsäure oder deren Salzen 2-80 Gewichtsprozent aufweist, und
- das Gewichtsverhältnis zwischen Phospholipiden und der Glycyrrhizinsäure oder deren Salze von 30:1 bis 0,5:1 beträgt,
zur Herstellung eines Arzneimittels zur therapeutischen Behandlung von subkutanen Fettgewebserkrankungen.

2. Verwendung gemäß Anspruch 1 zur Zersetzung und Rückbildung von Fettgewebsgeschwülsten.

3. Verwendung gemäß Anspruch 1, **dadurch gekennzeichnet, dass** es sich bei den subkutanen Fettgewebserkrankungen um Lipödeme, Lipome, Morbus Dercum, Madelung'schen Fetthals oder Lipomatosis der Bauchdecke handelt.

4. Nicht-therapeutische Verwendung einer Zusammensetzung, enthaltend
a) mindestens ein Phospholipid;
b) Glycyrrhizinsäure oder
ein Salz der Glycyrrhizinsäure und
c) ggf. Hilfsstoffe
wobei
- der Gesamtgehalt an den Phospholipiden und der Glycyrrhizinsäure oder deren Salzen 2-80 Gewichtsprozent aufweist, und
- das Gewichtsverhältnis zwischen Phospholipiden und der Glycyrrhizinsäure oder deren Salze von 30:1 bis 0,5:1 beträgt,
zur Behandlung von subkutanen Fettansammlungen.

5. Verwendung gemäß Anspruch 4 zur Entfernung von subkutanen Fettansammlungen, insbesondere mit lokaler Störung der Fettverteilung.

6. Verwendung gemäß Anspruch 5, **dadurch gekennzeichnet, dass** es sich bei der lokalen Störung der Fettverteilung um Dermatopanniculosis deformans, Pseudogynäkomastie, Buffalo Hump bei HIV Patienten, Cellulite, Xanthelasmen oder unspezifische subkutane Fettdepots handelt.

7. Verwendung gemäß irgendeinem der Ansprüche 1 bis 6, wobei die Zusammensetzung Phosphatidylcholin als Phospholipid enthält.

8. Verwendung gemäß irgendeinem der Ansprüche 1 bis 7, wobei die Zusammensetzung Phosphatidylcholin tierischen oder pflanzlichen Ursprungs enthält.

9. Verwendung gemäß irgendeinem der Ansprüche 1 bis 8, wobei die Zusammensetzung Glycyrrhizinsäure oder Kalium-, Natrium-, Ammonium- oder Magnesiumsalze der Glycyrrhizinsäure enthält.

10. Verwendung gemäß irgendeinem der Ansprüche 1 bis 9, wobei die Zusammensetzung einen Zucker, insbesondere Glukose und Maltose , Mannit, Sorbit oder Milchzucker als Hilfsstoff enthält.

11. Verwendung gemäß irgendeinem der Ansprüche 1 bis 10, wobei das Phospholipid 15 bis 98 Gew.-%, bevorzugt, 30 bis 98 Gew.-%, mehr bevorzugt 50 bis 98 Gew.-%, besonders bevorzugt 75 bis 98 Gew.-%, am meisten bevorzugt 75 bis 90 Gew.-% Phosphatidylcholin enthält.

12. Verwendung gemäß irgendeinem der Ansprüche 1 bis 11, wobei die Zusammensetzung in trockener Form in einem geeigneten Lösungsmittel gelöst wird.

13. Verwendung gemäß einem oder mehrerer der Ansprüche 1 bis 12, wobei die Zusammensetzung in trockener Form bevorzugt als Lyophilisat, erzielt durch Gefriertrocknung, eingesetzt wird.

14. Verwendung gemäß einem oder mehreren der Ansprüche 1 bis 13, wobei die Zusammensetzung in Form von einer Lösung eingesetzt wird.

15. Verwendung gemäß einem oder mehrerer der Ansprüche 1 bis 14, wobei die Zusammensetzung physiologisch geeignete Lösungsmittel umfassend Wasser, physiologische Kochsalzlösung, Glukose-Lösung, ein Monohydroxy-Alkohol wie Ethanol, 2-Propanol, n-Propanol, Polyhydroxy-Alkohole wie Glyzerol und/oder Propandiol, Polyglykol wie Polyethylenglykol und/oder Miglyol, Glycerinformal, Dimethylisosorbitol, natürliche und synthetische Öle und/oder Äther enthält.

16. Verwendung gemäß einem oder mehrerer der Ansprüche 1 bis 15, **dadurch gekennzeichnet, dass** die Applikation der Zusammensetzung durch subkutane, intraperitoneale, intramuskuläre oder intravenöse Injektion erfolgt.

17. Verwendung gemäß einem oder mehrerer der Ansprüche 1 bis 15, **dadurch gekennzeichnet, dass** zur Applikation der Zusammensetzung ein Verfahren ausgewählt aus der Gruppe umfassend lontophorese, Elektroporation, Microporation oder Phonophorese eingesetzt wird.

## Claims

1. Use of a composition, containing
a) at least one phospholipid;
b) glycyrrhizic acid or a salt of glycyrrhizic acid and
c) optionally excipients,
wherein
- the total content of phospholipids and glycyrrhizic acid or salts thereof is 2 - 80 percent by weight, and
- the weight ratio of phospholipids and glycyrrhizic acid or salts thereof is 30:1 to 0.5:1,
for manufacturing a medicament for therapeutic treatment of subcutaneous adipose tissue diseases.

2. Use according to claim 1 for degradation and regression of adipose tissue growth.

3. Use according to claim 1, **characterized in that** the subcutaneous adipose tissue diseases are lipoedema, lipoma, Morbus Dercum, Madelung's fat neck or lipomatosis of the abdominal wall.

4. Non-therapeutic use of a composition, comprising
a) at least one phospholipid;
b) glycyrrhizic acid or a salt of glycyrrhizic acid and
c) optionally excipients,
wherein
- the total content of phospholipids and glycyrrhizic acid or salts thereof is 2 - 80 percent by weight, and
- the weight ratio of phospholipids and glycyrrhizic acid or salts thereof is 30:1 to 0.5:1,
for treatment of subcutaneous fat accumulations.

5. Use according to claim 4 for removal of subcutaneous fat accumulations, in particular with local disorders of fat distribution.

6. Use according to claim 5, **characterized in that** the local disorder of fat distribution are dermatopanniculosis deformans, pseudogynecomastia, buffalo hump in HIV patients, cellulitis, xanthelasma or unspecific subcutaneous fat deposits.

7. Use according to any of claims 1 to 6 wherein the composition contains phosphatidylcholine as phospholipid.

8. Use according to any of claims 1 to 7 wherein the composition contains phosphatidylcholine of animal or vegetable origin.

9. Use according to any of claims 1 to 8 wherein the composition comprises potassium, sodium, ammonium or magnesium salts of glycyrrhizic acid.

10. Use according to any of claims 1 to 9 wherein the composition contains a sugar, in particular glucose and maltose, mannit, sorbit or lactose as excipient.

11. Use according to any of claims 1 to 10 wherein the phospholipid contains 15 to 98 percent by weight, preferably 30 to 98 percent by weight, more preferably 50 to 98 percent by weight, particularly preferred 75 to 98 percent by weight and most preferably 75 to 90 percent by weight of phosphatidylcholin.

12. Use according to any of claims 1 to 11 wherein the composition in dry form is dissolved in a suitable solvent.

13. Use according to one or more of claims 1 to 12 wherein the composition is used in dry form, preferably a lyophilisate obtained by freeze drying.

14. Use according to one or more of claims 1 to 13 wherein the composition is used in form of a solution.

15. Use according to one or more of claims 1 to 14 wherein the composition contains physiologically acceptable solvents comprising water, physiological saline, glucose-solution, a monohydroxy alcohol such as ethanol, 2-propanol, n-propanol, polyhydroxy alcohols such as glycerin and/or propandiol, polyglycol such as polyethylenglycol and/or Miglyol, glycerinformal, dimethylisosorbitol, natural or synthetic oils and/or ethers.

16. Use according to one or more of claims 1 to 15, **characterized in that** the application of the composition is carried out by subcutaneous, intraperitoneal, intramuscular or intravenous injection.

17. Use according to one or more of claims 1 to 15, **characterized in that** for application of the composition a method selected from the group comprising iontophoresis, electroporation, microporation or phonophoresis is used.

## Revendications

1. Utilisation d'une composition, contenant
a) au moins un phospholipide ;
b) de l'acide glycyrrhinique ou
un sel de l'acide glycyrrhinique et
c) en cas échéant des adjuvants
dans laquelle
- la teneur totale des phospholipides et de l'acide glycyrrhinique ou des sels de celui-ci est 2-80 % en poids, et
- le rapport pondéral entre les phospholipides et l'acide glycyrrhinique ou les sels de celui-ci est de 30 : 1 à 0,5 : 1,
pour la fabrication d'un médicament pour le traitement thérapeutique des maladies de tissus adipeux sous-cutanés.

2. Utilisation selon la revendication 1 pour la décomposition et la régression de grosseurs de tissus adipeux sous-cutanés.

3. Utilisation selon la revendication 1, **caractérisée par le fait que** les maladies de tissus adipeux sous-cutanés sont les lipoedèmes, les lipomes, la maladie de Dercum, le cou adipeux de Madelung ou la lipomatose de la paroi abdominale.

4. Utilisation non-thérapeutique d'une composition, contenant
a) au moins un phospholipide ;
b) de l'acide glycyrrhinique ou
un sel de l'acide glycyrrhinique et
c) en cas échéant des adjuvants
dans laquelle
- la teneur totale des phospholipides et de l'acide glycyrrhinique ou des sels de celui-ci est 2-80 % en poids, et
- le rapport pondéral entre les phospholipides et l'acide glycyrrhinique ou les
sels de celui-ci est de 30 : 1 à 0,5 : 1,
pour le traitement des accumulations adipeuses sous-cutanées.

5. Utilisation selon la revendication 4 pour l'enlèvement des accumulations adipeuses sous-cutanées, particulièrement avec les troubles locales de la distribution adipeuse.

6. Utilisation selon la revendication 5, **caractérisée par le fait que** le trouble locale de la distribution adipeuse est la Dermatopanniculosis deformans, la pseudogynécomastie, le buffalo Hump chez les patients de VIH, la cellulite, les xanthélasmes ou les dépôts adipeux sous-cutanés non-spécifiques.

7. Utilisation selon l'une quelconque des revendications 1 à 6, dans laquelle la composition contient de la phosphatidylcholine en tant que le phospholipide.

8. Utilisation selon l'une quelconque des revendications 1 à 7, dans laquelle la composition contient de la phosphatidylcholine d'origine des animaux ou d'origine végétal.

9. Utilisation selon l'une quelconque des revendications 1 à 8, dans laquelle la composition contient de l'acide glycyrrhinique ou des sels de potassium, sodium, ammonium ou de magnésium de l'acide glycyrrhinique.

10. Utilisation selon l'une quelconque des revendications 1 à 9, dans laquelle la composition contient un sucre, particulièrement le glucose et le maltose, le mannitol, le sorbitol ou le sucre de lait en tant que l'adjuvant.

11. Utilisation selon l'une quelconque des revendications 1 à 10, dans laquelle le phospholipide contient 15 % à 98 % en poids, préférablement 30 à 98 % en poids, plus préférablement de 50 à 98 % en poids, particulièrement préférablement 75 à 98 % en poids, et le plus préférablement 75 à 90 % en poids de la phosphatidylcholine.

12. Utilisation selon l'une quelconque des revendications 1 à 11, dans laquelle la composition est solubilisée en forme sèche dans un solvant approprié.

13. Utilisation selon l'une quelconque des revendications 1 à 12, dans laquelle la composition est utilisée en forme sèche préférablement en tant que lyophilisat, obtenu par lyophilisation.

14. Utilisation selon l'une quelconque des revendications 1 à 13, dans laquelle la composition est utilisée en forme d'une solution.

15. Utilisation selon l'une quelconque des revendications 1 à 14, dans laquelle la composition contient des solvants physiologiquement appropriés, comprenant de l'eau, la solution physiologique de sel, la solution de glucose, un alcool monohydroxylique tel que l'éthanol, le 2-propanol, le n-propane, les alcools polyhydroxyliques, tel que le glycérol et/ou le propandiol, le polyglycol, tel que le polyéthylène glycol et/ou le miglycol, le formiate de glycérol, le diméthylisosorbitol, les huiles naturelles et synthétiques et/ou l'éther.

16. Utilisation selon l'une quelconque des revendications 1 à 15, **caractérisée par le fait que** l'application de la composition est faite par injection sous-cutanée, intrapéritonéale, intramusculaire ou intraveineuse.

17. Utilisation selon l'une quelconque des revendications 1 à 15, **caractérisée par le fait qu'**un procédé sélectionné parmi le groupe comprenant l'ionotophorèse, l'électrolocation, la microporation ou la phonophorèse est utilisé pour l'application de la composition.
